# EUROPEAN PATENT SPECIFICATION

(11) **EP 4 342 905 B1**
(45) Date of publication and mention of the grant of the patent: **04.03.2026**
(21) Application number: 22197370.4
(22) Date of filing: 23.09.2022
(51) Int. Cl.: C07H 19/167, A61P 35/00

(54) **1,N6-ETHENOADENOSINE DERIVATIVES FOR CANCER TREATMENT**
1-N6-ETHENOADENOSINDERIVATE ZUR KREBSBEHANDLUNG
DÉRIVÉS DE LA 1,N6-ÉTHÉNOADÉNOSINE POUR LE TRAITEMENT DU CANCER

(43) Date of publication of application: 27.03.2024
(73) Proprietor: PHILIPPS-UNIVERSITÄT MARBURG, 35037 Marburg (DE); Universität Osnabrück, 49074 Osnabrück (DE)
(72) Inventor: Kinscherf, Ralf, 69198 Schriesheim (DE); Rosemeyer, Helmut, deceased (DE); Bonaterra, Gabriel A., Marburg 35039 (DE); Lünswilken, Mona, 35039 Marburg (DE); Bender, Eugenia, 49086 Osnabrück (DE); Barakat, Fatima, 30952 Ronnenberg (DE); Hammerbacher, Katharina, 67165 Mannheim (DE); Teusch, Nicole, 50859 Köln (DE); Bartsch, Jörg-Walter, 33617 Bielefeld (DE); Bernert, Marco, 69517 Gorxheimertal (DE)
(74) Representative: Durm Patentanwälte PartG mbB

(56) References cited:
- HAMMERBACHER KATHARINA ET AL: "Combinatorial Synthesis of New Pyrimidine- and Purine-[beta]-D-Ribonucleoside Nucleolipids: Their Distribution Between Aqueous and Organic Phases and Their In Vitro Activity Against Human- and Rat Glioblastoma Cells In Vitro", CHEMISTRY & BIODIVERSITY, vol. 15, no. 9, 21 June 2018 (2018-06-21), CH, pages e1800173, XP093023113, ISSN: 1612-1872, Retrieved from the Internet <URL:https://api.wiley.com/onlinelibrary/tdm/v1/articles/10.1002%2Fcbdv.201800173> DOI: 10.1002/cbdv.201800173

## Description

The invention relates to adenosine and 1,N⁶-ethenoadenosine derivatives which are suitable for the treatment of cancer such as gliomas, glioblastomas, colon adenocarcinomas, hepatocellular carcinomas, renal carcinomas, pancreatic carcinomas and pancreatic adenocarcinomas.

Cancer is one of the leading causes of death in the world. Chemotherapeutic standard treatments include the administration of temozolomide (TMZ) for brain tumors, and 5-fluorouracil (5-FU) or 5-fluorouridine (5-FUrd) for e.g. colon and stomach cancers. However, the compounds currently used for chemotherapy are not satisfactory, and there is a continuing need for new compounds.

Hammerbacher et al., Chem. Biodiversity 2018, 15, e1800173, demonstrated that pyrimidine and purine-β-D-ribonuclosides with symmetric ketal groups at the O-2',3' position of the gly-conic moiety and farnesyl groups at N(3) of pyrimidines or N(1) of purines show partly pronounced *in vitro* cytostatic/cytotoxic activity toward rat-specific glioma cells.

C. Knies, Nucleolipide: Synthese und Biomedizinische Aspekte, Dissertation, Universität Osnabrück, 2017, found that ketalization of adenosine with undecane-6-one or nonadecane-10-one gave adenosine derivatives which were active against rat malignant neuroectodermal BT4Ca and human GOS3 glioma cells.

Despite these promising results, the currently known compounds are not sufficient, and suitable alternative therapies are not yet available, so alternative therapies are needed.

DE 10 2004 009 977 A1 discloses fluorescent ethenonucleosides and oligonucleotides with high stability.

Barrio et al., Biochemical and Biophysical Research Communications 1972, 46, 597, describe the synthesis of the fluorescent 1,N⁶-ethenoadenosine and 3,N⁴-ethenocytidine by reaction of adenosine and cytidine, respectively, with chloroacetaldehyde under mildly acidic conditions.

It is an object of the present invention to provide new compounds for the treatment of cancer that have high anti-cancer activity and a high therapeutic index.

This object is achieved by adenosine derivatives of Formula I, or pharmaceutically acceptable salts thereof: 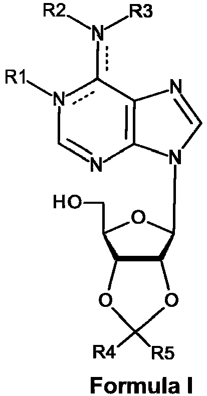 wherein:
R¹ and R² together form an ethenylene group, the bond between N¹ and C⁶ is a single bond, the bond between C⁶ and the exocyclic amino nitrogen is a double bond, and R³ is absent,
R⁴ and R⁵ are each independently a branched or preferably linear C₁-C₁₆-alkyl group, preferably a C₁-C₁₅-alkyl group, more preferably a C₁-C₁₃ alkyl group, provided that R⁴ and R⁵ together comprise 11 to 17, preferably 11 to 16 carbon atoms, or R⁴ and R⁵ together with the carbon atom to which they are bound form a monocyclic ring with 10 to 15, preferably 10 to 14, carbon atoms or a polycyclic cycloaliphatic ring with 10 to 15, preferably 10 to 14, carbon atoms.

Any compound, general structure, composition, or use disclosed hereinafter which does not fall within the scope of the appended claims is intended as a reference and does not constitute an embodiment according to the invention.

The atoms of the heterocyclic purine ring system are numbered as follows:

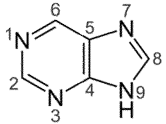

All formulas shown herein extend only to those compounds which are compatible with the theory of chemical valence and encompass all stereoisomeric, tautomeric and anomeric forms. The dashed lines in Formula I indicate that the respective bonds are a single bond or a double bond, where one bond is a single bond and the other bond is a double bond.

Preferred are adenosine derivatives of Formula I are compounds wherein both R⁴ and R⁵ are each a linear C₆-C₈ alkyl group, preferably a C₇ to C₈ alkyl group, or one of R⁴ and R⁵ is a linear C₁-C₃-alkyl group, preferably C₁-C₂-alkyl group, and the other is a linear C₈-C₁₆ alkyl group, preferably C₁₀-C₁₅ alkyl group, more preferably a C₁₃ alkyl group. Particularly preferred compounds with linear alkyl groups are represented by Formula II: 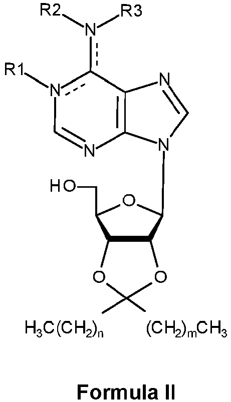 wherein:
n and m are each an integer of 0 to 15, preferably 0 to 14, more preferably 0 to 12, provided that the sum of m and n is in a range of from 9 to 15, preferably 10 to 14, more preferably 11 to 14 and most preferably 12 to 14.

Particularly preferred compounds of Formula II are compounds wherein m=n= 6; m=n=7; n=0 and m=12; n=1 and m=12.

In compounds with an unsymmetrical O-2',3' ketal group, the ketalic carbon atom is asymmetric and two enantiomers exist, the R enantiomer and the S enantiomer. According to one embodiment, the invention relates to the 1S enantiomers of Formula I, and according to another embodiment to the 1R enantiomers of Formula I, with the R enantiomers being preferred. Symmetrical ketal groups are obtained, for example, if n and m are the same; if n and m are different, the ketal group is unsymmetrical.

According to another preferred embodiment of the invention, R⁴ and R⁵ together with the carbon atom to which they are bound form a polycyclic and preferably a monocyclic cycloaliphatic ring. Unsubstituted monocylclic ketal groups are symmetrical, while substituted cyclic groups can symmetrical or unsymmetrical. Compounds with monocyclic rings are preferred and are represented by Formula III: 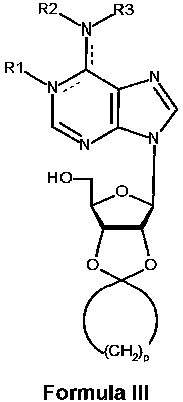 wherein p is an integer within the range of from 9 to 14, preferably 10 to14 and most preferably 10 to 13.

Formulas I to III encompass compounds wherein R¹ is absent, the bond between N¹ and C⁶ is a double bond, the bond between C⁶ and the amino nitrogen is a single bond, and R² and R³ are each H, and compounds wherein R¹ and R² together form an ethenylene group, the bond between N¹ and C⁶ is a single bond, the bond between C⁶ and the amino nitrogen is a double bond, and R³ is absent (1,N⁶-ethenoadenosine derivatives). These two alternatives are exemplified for Formula I. Formula la shows compounds to the first alternative and Formula Ib 1,N⁶-ethenoadenosine derivatives according to the second alternative, compounds according to the first alternative are reference compounds and do not constitute embodiments according to the invention:

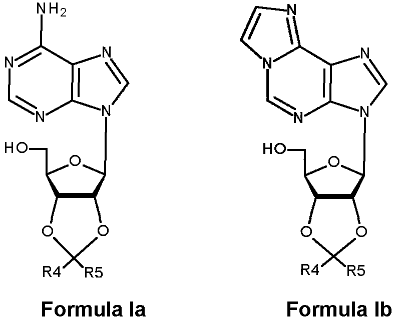

It follows from the above that adenosine derivatives of Formula la and Ib wherein both R⁴ and R⁵ are each a linear C₆-C₈ alkyl group, preferably a C₇ to C₈ alkyl group, or one of R⁴ and R⁵ is a linear C₁-C₂-alkyl group and the other is a linear C₁₁-C₁₅ group, preferably a C₁₃ group, are preferred. Compounds of Formula la are reference compounds and do not constitute embodiments according to the invention.

Particularly preferred compounds according to Formula la are:

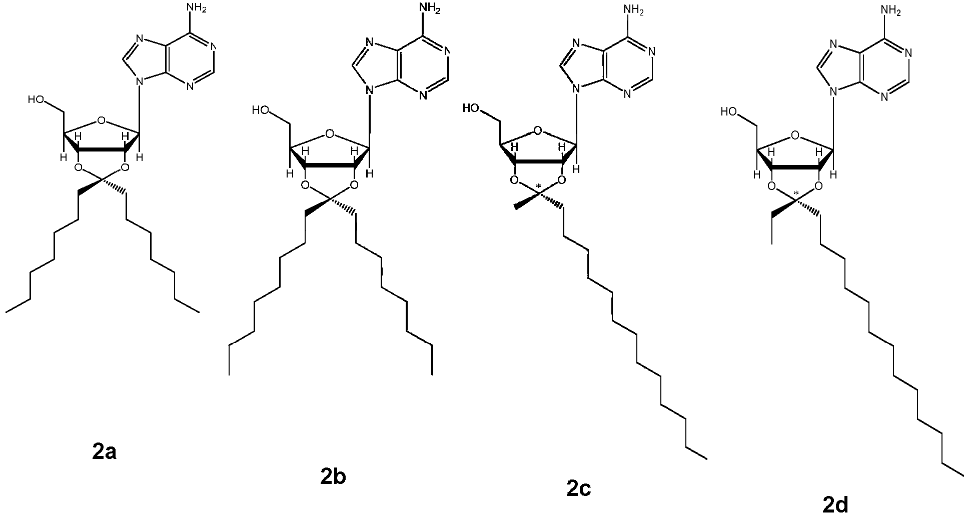

Particularly preferred compounds according to Formula Ib are:

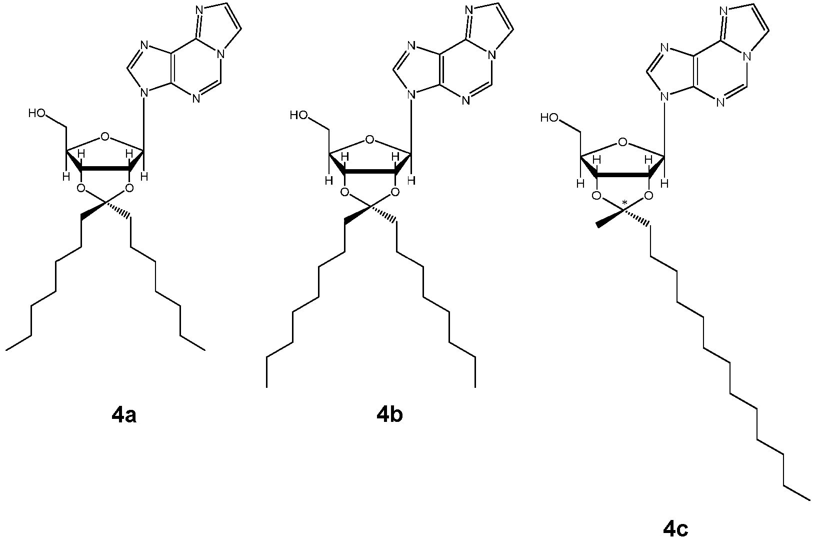

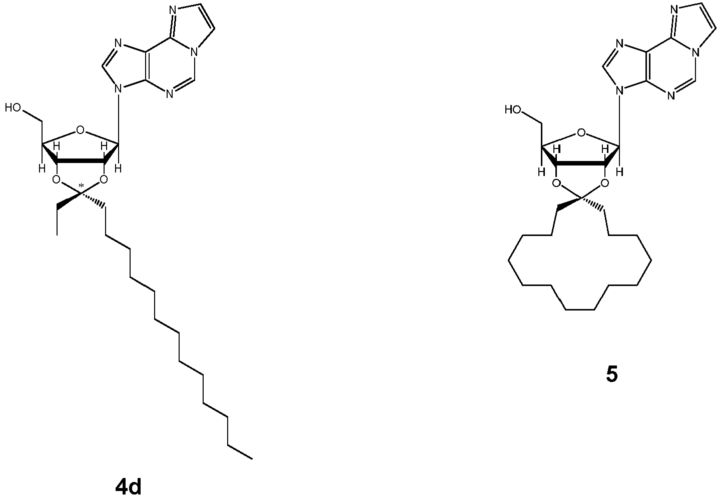

For compounds 2c, 2d, 4c and 4d, the R enantiomers are shown. However, the S enantiomers are also subject of the present invention, with the R enantiomers being preferred. The asymmetric carbon atoms are marked with an asterisk.

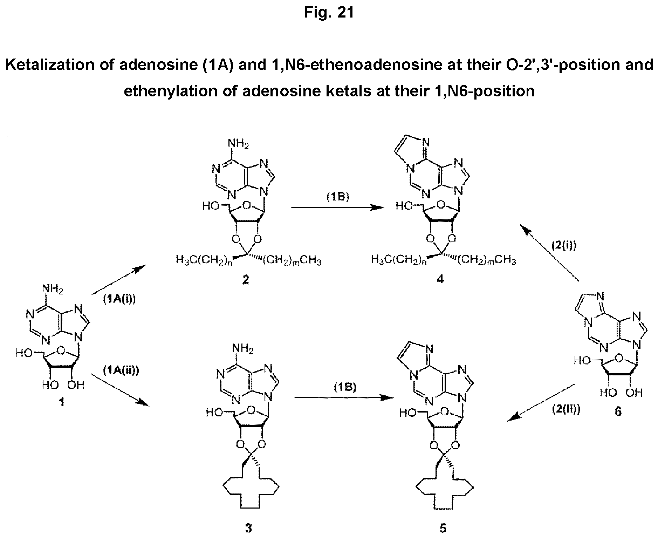

The compounds according to Formula I can be synthesized in analogy to known procedures, as exemplified in Figure 21. For instance, adenosine 1 can be ketalized at its O-2',3'-position by reaction with an orthoester, which leads to the formation of an acetal, followed by reaction with the ketone H₃C(CH₂)ₙC(=O)(CH₂)ₘCH₃ to obtain the desired ketal 2 (Figure 21, reaction 1A(i)). Ketal 2 can then be ethenylated at its 1,N⁶-position with chloroacetaldehyde to obtain the corresponding 1,N⁶-ethenoadenosine derivative 4 (reaction 1B). Alternatively, 1,N⁶-ethenoadenosine 6 can be formed first, which is then ketalized to 4 (reaction 2(ii)). Cyclic ketals according to Formula III can be obtained by reaction of adenosine 1 or 1,N⁶-ethenoadenosine 6 with cyclic ketones (reactions 1A(ii) and 2(ii)).

The tricyclic 1,N⁶-ethenoadenosine derivatives according to Formula Ib have fluorescent properties. They can be detected by excitation at a specific wavelength and can thus be made visible in biological materials by using appropriate measuring instruments. Due to their fluorescent properties, these compounds, in addition to their use in the treatment of cancer, can be used to study mechanisms of enzymatic reactions, to elucidate their biological mode of action and as diagnostic or therapeutic probes.

Surprisingly, the compounds of the above formulas were found to be highly active against various tumor cell lines and in particular various carcinomas. They have cancerostatic and cancerotoxic activity. The compounds according to Formula I are characterized by high therapeutic index. The therapeutic index, also referred to as therapeutic ratio, is a quantitative measurement of the relative safety of a drug. It is a comparison of the amount of a therapeutic agent that causes the therapeutic effect to the amount that causes toxicity.

According to the present invention, compounds of Formula I with a molecular weight of ≤ 530 g/mol and in particular ≤ 500 g/mol are preferred. More preferably, the molecular weight is within a range of from 470 to 530 g/mol, most preferably 470 to 500 g/mol.

The compounds of the present invention are characterized by an advantageous number of H-bridge donors, such as -NH₂ and -OH, and H-bridge acceptors, such as =O and =N-. Amino groups can form one or two hydrogen bridges. The number of H-bridge donors and acceptors influences the passive transport of the compounds across the cell membrane and an excessive number of H-bridge donors or acceptors may inhibit the passive transport across cell membranes. According to the invention, compounds comprising up to 5, preferably 1 to 3 and more preferably 1 or 2, H-bridge donors and/or up to 10, preferably 6 to 9 and more preferably 7 or 8, H-bridge acceptors are preferred.

Furthermore, compounds according to Formula I having a partition coefficient (logP_{OW}) of ≤ 5 are preferred. More preferably, the logP_{OW} coefficient is within a range of 1 to 5 and most preferably 1.5 to 4. The lipophilicity and thus the partition coefficient can be adjusted by the appropriate selection of the residues R¹ to R⁵.

All logP_{OW} values reported herein are determined as follows: 2 mg of the compound to be measured are distributed in a 250 ml beaker between a two-phase mixture of water (50 ml) and 1-octanol (50 ml) and stirred for 1 h on a magnetic stirrer at room temperature (23 °C). After separation of the layers, 3 ml of each phase are removed and transferred into a 3.5 ml quartz cuvette (10 x 10 mm, 3500 µl; Hellma, Darmstadt, Germany) and measured on a UV/VIS spectrometer (Cary 50, Varian, Darmstadt, Germany). From the ratio of maximal absorbances in both layers (λmax) the corresponding log₁₀P_{OW} values are determined.

According to the present invention, compounds which meet at least two of these conditions, i.e. molecular weight of 530 g/mol or less, up to 5 H-bridge donors, up to 10 H-bridge acceptors, logP_{OW} of ≤ 5, are particularly preferred, with compounds meeting at least three conditions being more preferred and compound meeting all four conditions being most preferred. Naturally, substances in which the values lie in the preferred and particularly preferred ranges are especially preferred. It was found that compounds which meet at least two, preferably at least three and most preferably all four conditions exhibit improved cell membrane permeability, improved passive transport across cell membranes, and improved cellular absorption.

The molecular weights, number of H-bridge donors and acceptors and the logP_{OW} values of particularly preferred compounds of the present invention are summarized in the following table:

| **Compound** | **Molecular Weight [g/mol]** | **H-bridge Donors** | **H-bridge Acceptors** | **log*P*_{*OW* (measured)}** |
|---|---|---|---|---|
| **2a** | 475.63 | 2 | 7 | 2.82 |
| **2b** | 503.69 | 2 | 7 | 3.86 |
| **2c** | 419.53 | 2 | 7 | 2.83 |
| **2d** | 475.63 | 2 | 7 | 3.60 |
| **4a** | 499.66 | 1 | 8 | 2.40 |
| **4b** | 527.71 | 1 | 8 | 2.57 |
| **4c** | 499.66 | 1 | 8 | 2.75 |
| **4d** | 513.33 | 1 | 8 | 2.29 |
| **5** | 497.64 | 1 | 8 | 1.67 |

The Formula I compounds were shown to have cytotoxic activity on rat malignant neuroectodermal BT4Ca and human GOS3 glioma cells, human U87, U251, G112 and G28 glioblastoma cells, human HT29 colon adenocarcinoma cells, human HepG2 hepatocellular carcinoma cells, murine RenCa renal carcinoma cells, and human Panc-1 pancreatic carcinoma cells.

The *in vitro* cytotoxic effects of compounds of the present invention on different types of cancer cells such as e. g. glioma, glioblastoma, colon adenocarcinoma, pancreatic ductal adenocarcinoma, hepatocellular carcinoma, renal carcinoma, and pancreatic carcinoma, open up a wide therapeutic spectrum for chemotherapeutic treatment of different kinds of cancer. The invention relates to a class of adenosine and 1,N⁶-ethenoadenosine derivatives with improved efficacy on certain types of cancer compared to TMZ, 5-FU and 5-FUrd. It was found that the introduction of specific symmetric, asymmetric or cyclic ketal groups at the O-2',3' position of the glycone moiety results in pronounced cytostatic/cytotoxic activity towards different cancer entities of various species *in vitro.*

When comparing the cytostatic/cytotoxic effects with known anti-cancer drugs such as TMZ, or 5-FU, the compounds of Formula I are more efficacious. The efficacy of the compounds according to the invention is higher than that of TMZ or 5-FU, i.e., the compounds are more efficacious than TMZ even at concentrations 10 times lower. Moreover, unlike the compounds of Formula I, 5-FU never achieved 100% cytotoxicity at any concentration tested. For all these reasons, the compounds of Formula I are expected to have fewer side effects than TMZ or 5-FU when used *in vivo.*

The compounds of Formula I, in particular compounds 2a, 2d, 4b and 4c, were even found to be more active against human Panc-1 pancreatic carcinoma cells than gemcitabine, the current standard drug for treating pancreatic carcinomas.

The compounds of Formula I, or a pharmaceutically acceptable salt thereof, are suitable for the treatment of cancer. They are especially suitable for the therapy of brain cancer, such as malignant brain tumors, e.g. gliomas and especially glioblastomas, and for the therapy of carcinomas. The compounds of Formula I are particularly suitable for the treatment of colon cancer, especially adenocarcinomas of the colon, liver cancer, especially hepatocellular carcinomas, renal cancer, especially renal cell carcinomas, pancreatic cancer, especially pancreatic carcinomas and adenocarcinomas, such as pancreatic ductal adenocarcinomas.

The present invention also relates to pharmaceutical compositions comprising a pharmaceutically effective amount of at least one compound according to Formula I, or a salt thereof, for use in the treatment of cancer. The pharmaceutical compositions preferably also comprise at least one pharmaceutically acceptable excipient, preferably a liquid carrier. The pharmaceutical composition is preferably a liquid composition, more preferably an aqueous composition. Injectable compositions are preferred, in particular compositions for parenteral administration. For example, the compounds can be provided in the form of a dry powder for reconstitution with water for injection, preferably with water comprising 0.9% by weight of NaCl or 5% by weight of glucose.

In the following the invention will be described in more detail with reference to figures and examples.

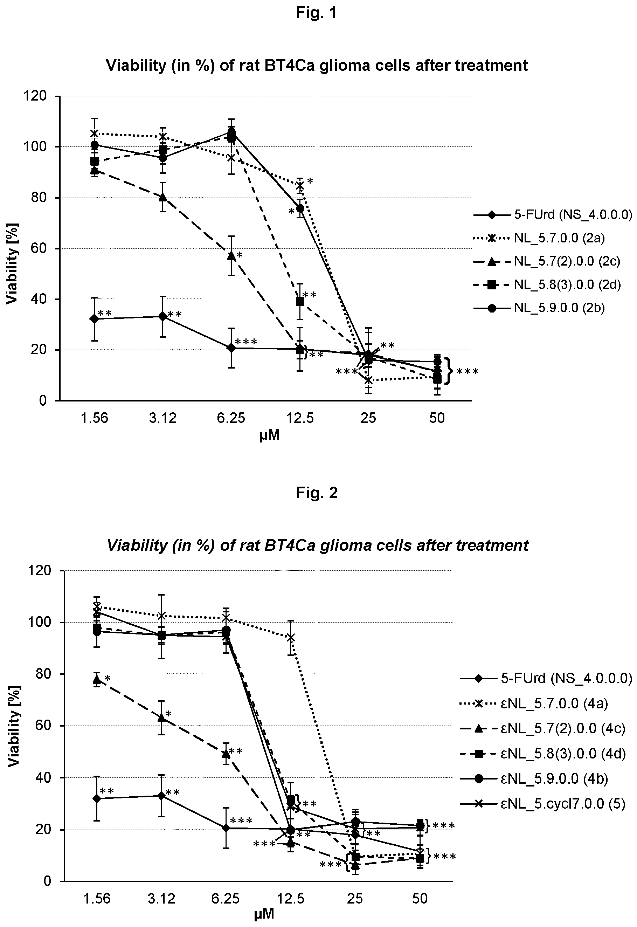

**Figures 1 and 2** show the *in vitro* activity of compounds 2a-d, 4a-d and 5 according to the present invention against rat malignant neuroectodermal BT4Ca glioma cells compared to 5-FUrd. It can be seen that all compounds are highly active at a concentration of 25 µM and that many compounds are active already at 12.5 µM.

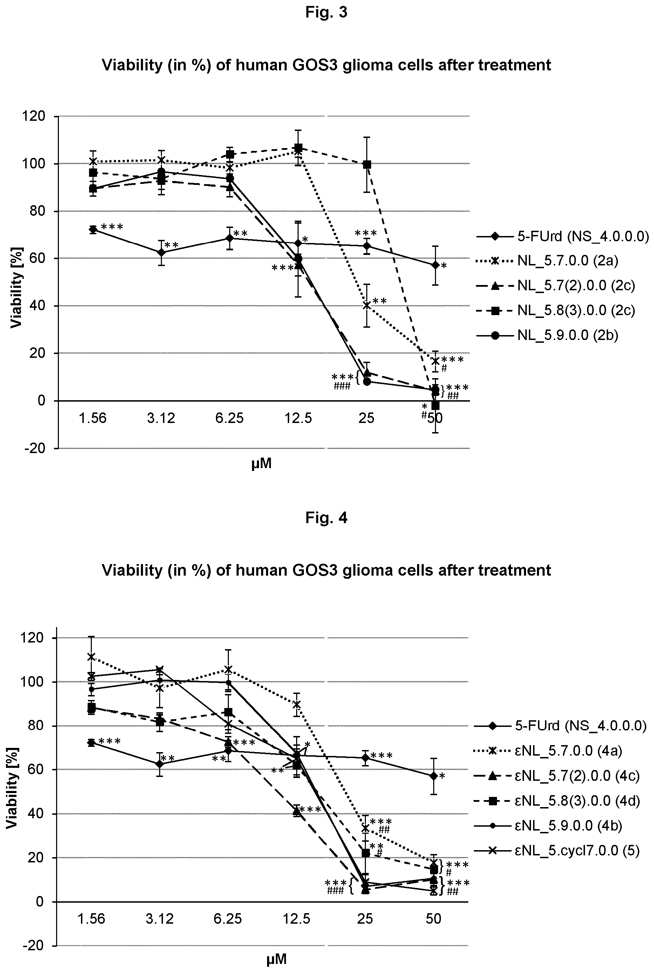

**Figures 3 and 4** show the *in vitro* activity of compounds 2a-d, 4a-d and 5 according to the present invention against human GOS3 glioma cells compared to 5-FUrd. It can be seen that all compounds are highly active at a concentration of 50 µM and that many compounds are active already at 12.5 µM or 25 µM. At 50 µM, all compounds are more active than 5-FUrd, the current standard drug for treating gliomas.

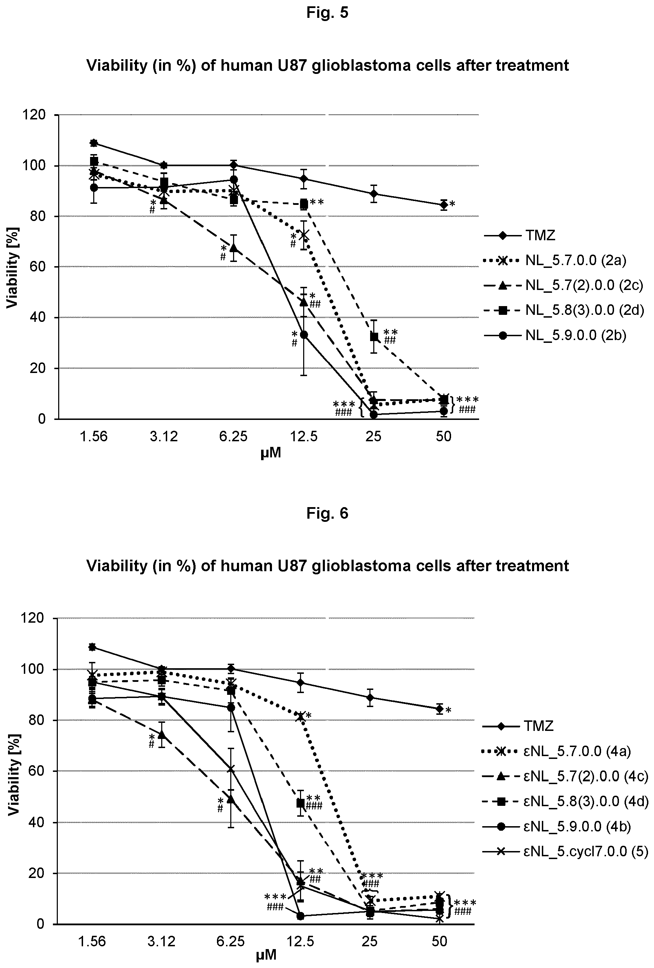

**Figures 5 and 6** show the *in vitro* activity of compounds 2a-d, 4a-d and 5 according to the present invention against human U87 glioblastoma cells compared to TMZ. It can be seen that all compounds are highly active at a concentration of 25 µM and that many compounds are active already at 12.5 µM. At all concentrations tested, the compounds according to the invention are more active than TMZ, the current standard drug for treating glioblastomas.

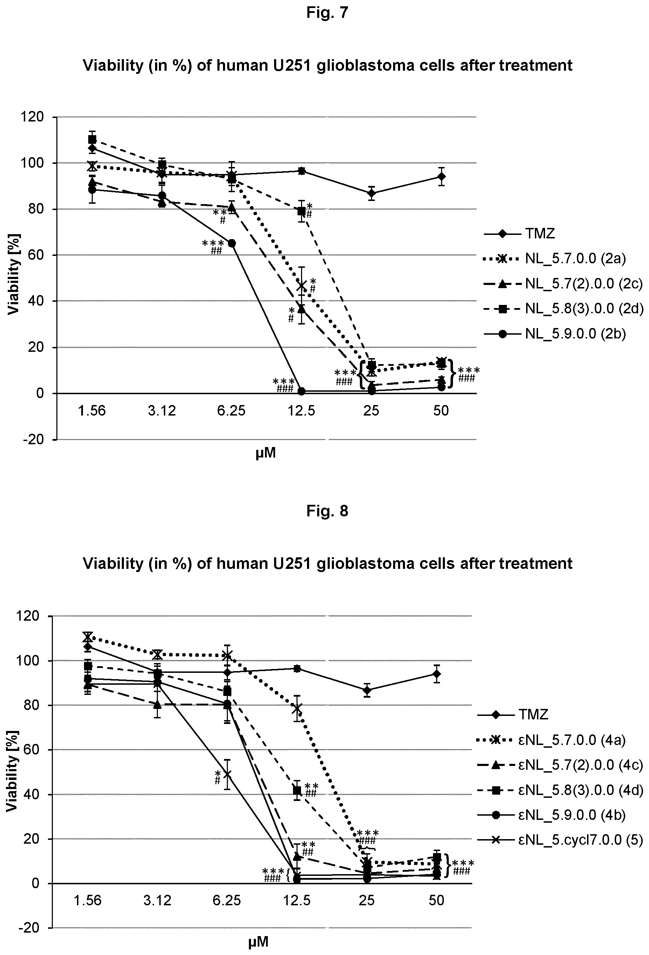

**Figures 7 and 8** show the *in vitro* activity of compounds 2a-d, 4a-d and 5 according to the present invention against human U251 glioblastoma cells compared to TMZ. It can be seen that all compounds are highly active at a concentration of 25 µM and that many compounds are active already at 12.5 µM. The compounds according to the invention are more active than TMZ, the current standard drug for treating glioblastomas.

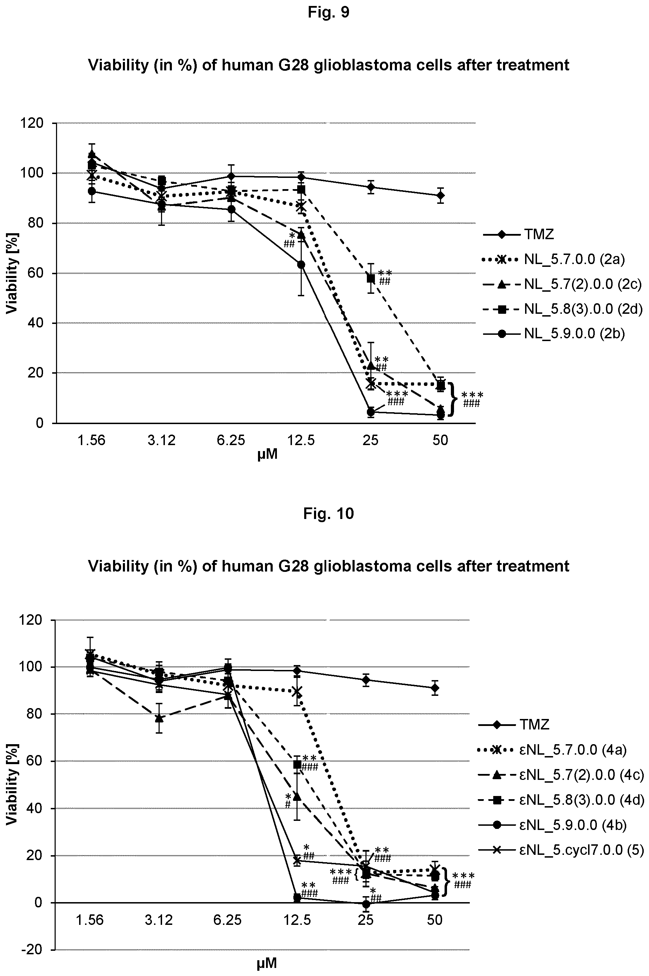

**Figures 9 and 10** show the *in vitro* activity of compounds 2a-d, 4a-d and 5 according to the present invention against human G28 glioblastoma cells compared to TMZ. It can be seen that all compounds are highly active at a concentration of 25 µM and that many compounds are active already at 12.5 µM. The compounds according to the invention are more active than TMZ, the current standard drug for treating glioblastomas.

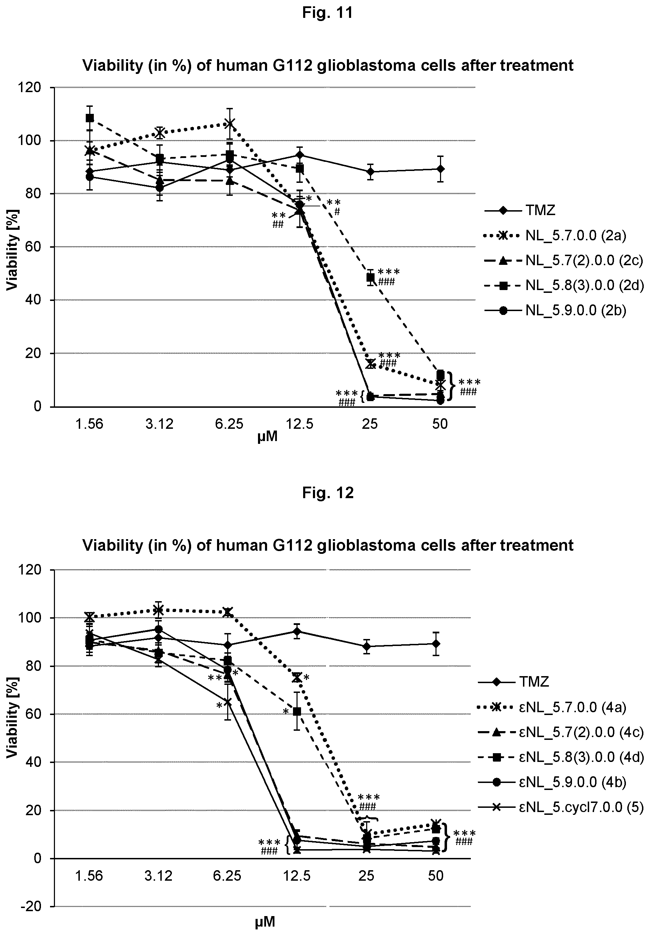

**Figures 11 and 12** show the *in vitro* activity of compounds 2a-d, 4a-d and 5 according to the present invention against human U87 glioblastoma cells compared to TMZ. It can be seen that all compounds are highly active at a concentration of 50 µM and that many compounds are active already at 12.5 µM or 25 µM. The compounds according to the invention are more active than TMZ, the current standard drug for treating glioblastomas.

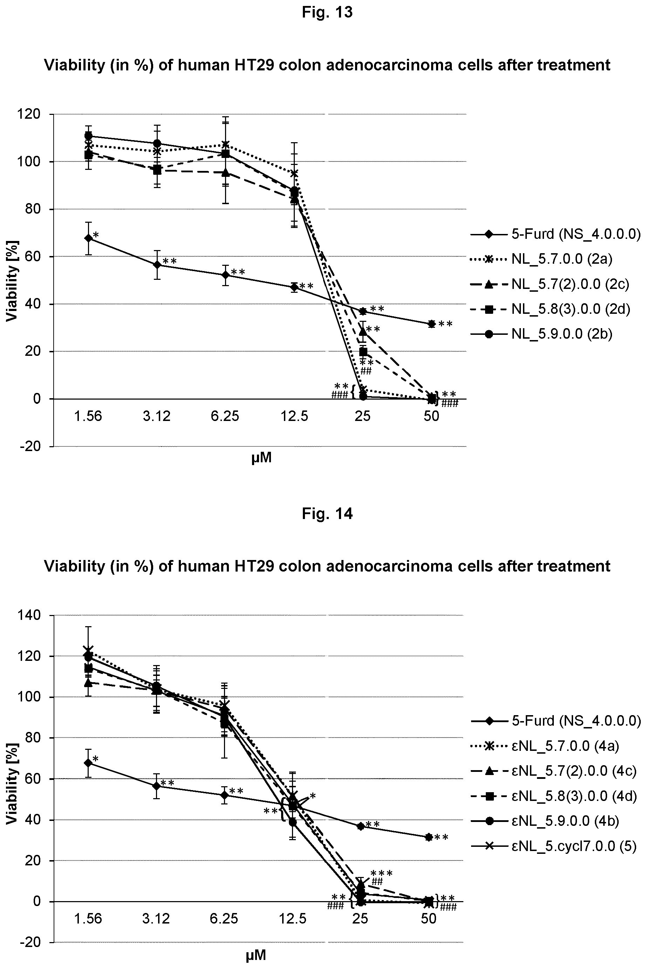

**Figures 13 and 14** show the *in vitro* activity of compounds 2a-d, 4a-d and 5 according to the present invention against human colon adenocarcinoma cells compared to 5-FUrd. It can be seen that all compounds are highly active at a concentration of 25 µM and that many compounds are active already at 12.5 µM. At concentrations of 25 µM or more, the compounds according to the invention are more active than 5-FUrd, the current standard drug for treating colon adenocarcinomas.

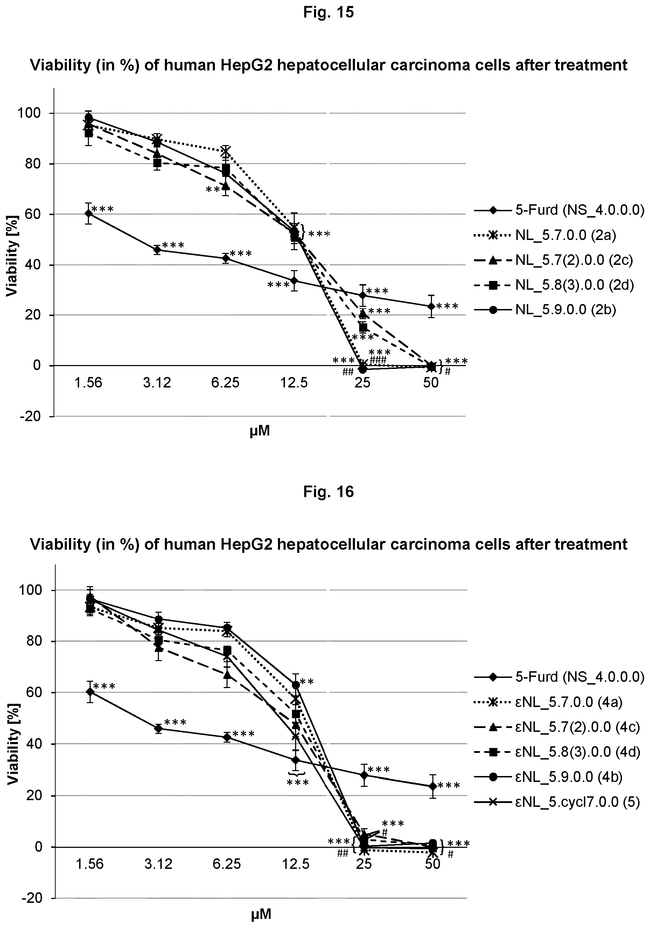

**Figures 15 and 16** show the *in vitro* activity of compounds 2a-d, 4a-d and 5 according to the present invention against human HepG2 hepatocellular carcinoma cells compared to 5-FUrd. It can be seen that all compounds are highly active at a concentration of 25 µM and that many compounds are active already at 12.5 µM. At concentrations of 25 µM or more, the compounds according to the invention are more active than 5-FUrd, the current standard drug for treating hepatocellular carcinomas.

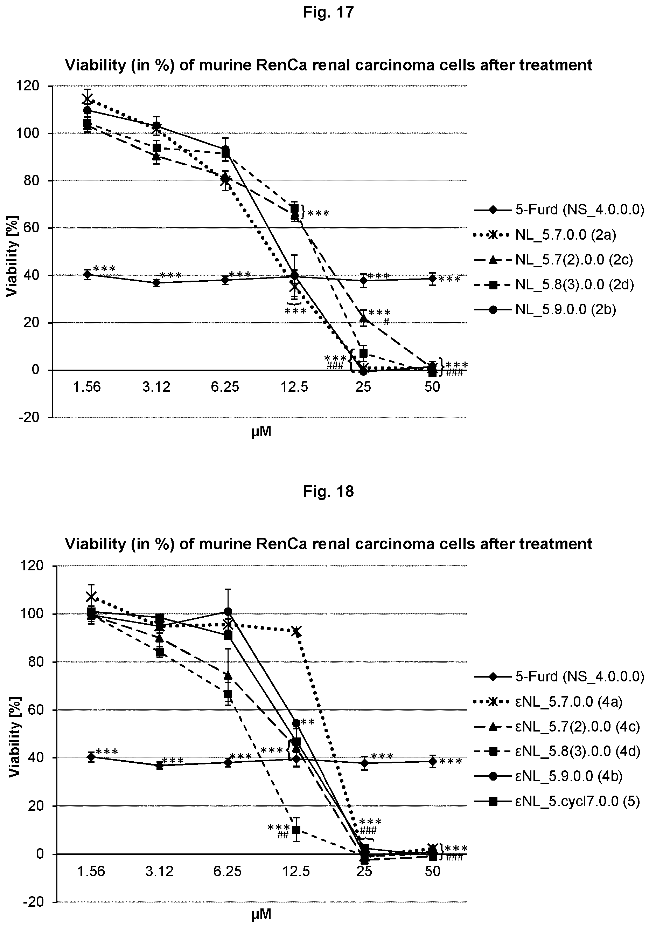

**Figures 17 and 18** show the *in vitro* activity of compounds 2a-d, 4a-d and 5 according to the present invention against murine RenCa renal carcinoma cells compared to 5-FUrd. It can be seen that all compounds are highly active at a concentration of 25 µM and that many compounds are active already at 12.5 µM. At concentrations of 25 µM or more, the compounds according to the invention are more active than 5-FUrd, the current standard drug for treating renal carcinomas.

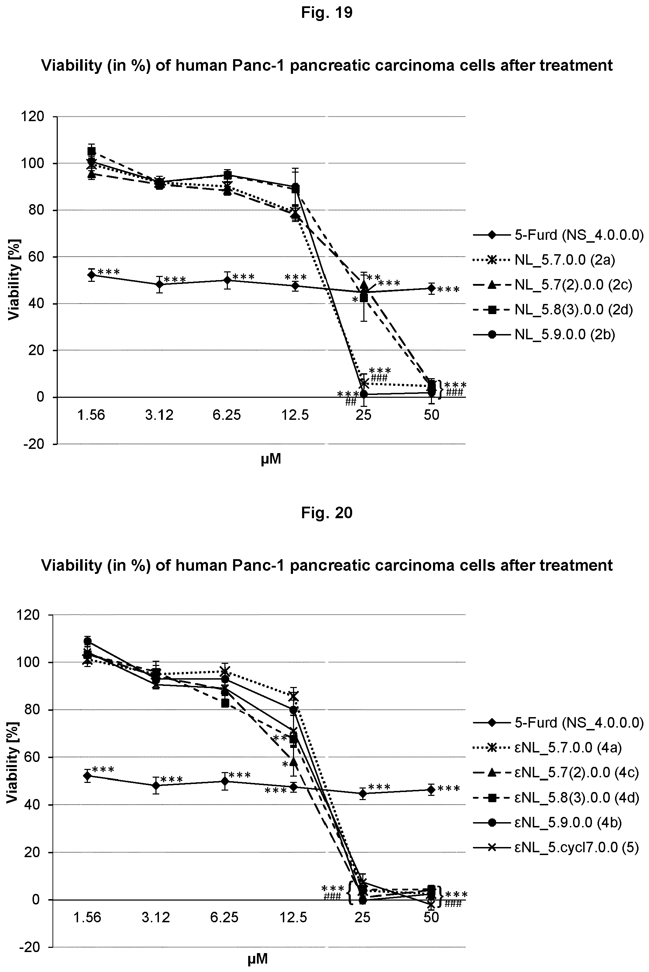

**Figures 19 and 20** show the *in vitro* activity of compounds 2a-d, 4a-d and 5 according to the present invention against human Panc-1 pancreatic carcinoma cells compared to 5-FUrd. It can be seen that all compounds are highly active at a concentration of 25 µM. At concentrations of 25 µM or more, the compounds according to the invention are more active than 5-FUrd.
**Figure 21** is a reaction scheme showing preferred synthetic pathways for the synthesis of compounds according to Formula I by ketalization of adenosine or 1,N⁶-ethenoadenosine at their O-2',3'-position or ethenylation of adenosine ketals at their 1,N⁶-position.

### Examples

### Reference Example 1

### ((3aR,4R,6R,6aR)-6-(6-amino-9H-purin-9-yl)-2,2-diheptyltetrahydrofuro[3,4-d][1,3]dioxol-4-yl)methanol (2a, NL 5.7.0.0)

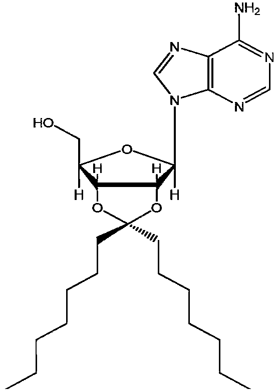

Anhydrous adenosine (1, NS_5.0.0.0; 1.0 g; 3.74 mmol) was dissolved in 15 ml of dry and amine-free dimethylformamide. Pentadecan-8-one (0.906 g; 4.0 mmol), triethyl orthoformate (0.662 ml) and 4M HCl in 1.4-dioxane (7.5 ml) were then added. The mixture was stirred at room temperature for 24 hours and then distributed between 60 ml of CH₂Cl₂ and a saturated aqueous solution of sodium hydrogen carbonate. The aqueous phase was extracted 3 times with 12.5 ml CH₂Cl₂. The combined organic phases were washed with 120 ml H₂O and then dried for 1 h over MgSO₄. The filtered raw product was concentrated at a rotary evaporator and evaporated several times with CH₂Cl₂ harshly to remove the remaining DMF. The raw product was dried overnight in high vacuum at room temperature. The resulting product was obtained by column chromatography on silica gel (SiO₂ 60, column: 6.5 cm x 7.5 cm; CH₂Cl₂/MeOH 9:1; v/v). The isolated product, freed from the solvent, forms a white solid in high vacuum (0.9562 g, 54%).

TLC (SiO₂ 60, CH₂Cl₂/MeOH 9:1; v/v): *R_{f}* 0.80. UV/Vis UV/Vis (MeOH): λ_{ma×}: 259 nm, ε=14648.0 l·mol⁻¹·cm⁻¹. log*P*_{OW}: 2.82. ¹H-NMR (500.13 MHz, DMSO - *d₆*): 8.335 (s, 1H, H-C(2)); 8.148 (s, 1H, H-C(8)); 7.281 (s, 2H, NH₂); 6.132 (d, 1H, ³J(H-C(1'), H-C(2')) =3.0, H-C(1')); 5.364 *(dd,* 1H, ³J(H-C(2'), H-C(1')) =3.0, ³J(H-C(2'), H-C(3')) =6.5, H-C(2')); 5.099 *(t,* 1H, ³J(HO-C(5'), H₂-C(5')) =5.5, HO-C(5')); 4.964 (dd, 1H, ³J(H-C(3'), H-C(2')) =2.5, ³J(H-C(3'), H-C(4')) =6.0, H-C(3')); 4.203 - 4.178 *(m,* 1H, H-C(4')); 3.558 - 3.460 *(m,* 2H, H₂-C(5')); 1.740 - 1.708 *(m,* 2H, H₂C(1a")); 1.562 - 1.546 *(m,* 2H, H₂-C(1b")); 1.432 - 1.423 *(m,* 2H, H₂-C(2a")); 1.311 - 1.243 *(m,* 18H, H₂-C(2b"), (H₂-C(3a"), (H₂-C(3b"), (H₂-C(4a"-6a"), (H₂-C(4b"-6b")); 0.884 - 0.825 *(m,* 6H, H₃-C(7a"), H₃-C(7b")). ¹³C-NMR (125.76 MHz, DMSO-*d₆*): 156.043 (C(6)); 152.527 (C(2)); 148.806 (C(4)); 139.613 (C8)); 118.978 (C(5)); 116.654 (C(acetal)); 89.410 (C(1')); 86.832 (C(4')); 83.542 (C(2')); 81.478 (C(3')); 61.518 (C(5')); 36.376 (C(1a")); 36.198 (C(1b")); 31.134 (C(2a")); 31.076 (C(2b")); 28.518 (C(3a")); 28.450 (C(3b")); 21.976 (C(4a"-6a")); 21.938 (C(4b"-6b")); 13.822 (C(7a")); 13.784 (C(7b")). ESI - MS: 476.26 [M_{W}+H]⁺ (calculated: M_{W}=475.62). Elemental analysis calculated for C₂₅H₄₁N₅O₄ · 0.15 H₂O: C, 62.95 (62.78); H, 8.89 (8.7); N, 14.36 (14.64).

### Reference Example 2

### ((3aR,4R,6R,6aR)-6-(6-amino-9H-purin-9-yl)-2,2-dioctyltetrahydrofuro[3,4-d][1,3]dioxol-4-yl)methanol (2b, NL 5.9.0.0)

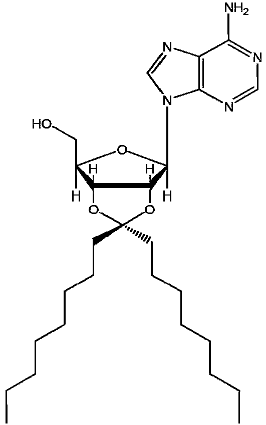

Dry adenosine (1, NS_5.0.0.0; 1 g, 3.74 mmol) was dissolved in 15 ml of dry and amine-free dimethylformamide. Heptadecan-9-one (1.018 g, 4 mmol), triethyl orthoformate (0.662 ml, 4 mmol) and 4M HCl in 1,4-dioxane (7.5 ml) were added. The mixture was stirred at room temperature for 24 hours and then distributed between 80 ml of CH₂Cl₂ and a saturated aqueous solution of sodium hydrogen carbonate. The aqueous phase was extracted 3 times with 12 ml CH₂Cl₂. The combined organic phases were washed with H₂O (3 x 50 ml) and then dried for 30 minutes over MgSO₄. The filtered raw product was concentrated at a rotary evaporator an evaporated several times with CH₂Cl₂ harshly to remove the remaining DMF. The raw product was dried overnight in high vacuum at room temperature. The resulting product was obtained by column chromatography on silica gel (SiO₂ 60, column: 6.5 cm x 11.5 cm; CH₂Cl₂/MeOH 9:1; v/v). The isolated product, freed from the solvent, forms a white solid in high vacuum (1.2997 g, 69 %).

TLC (SiO₂ 60, CH₂Cl₂/MeOH 9:1; v/v): *R_{f}* 0.82. UV/Vis (MeOH): λ_{ma×}: 260 nm, ε=13704.7 l·mol⁻¹·cm⁻¹. log*P*_{OW}: 3.86. ¹H-NMR (500.13 MHz, DMSO - *d*₆): 8.333 (s, 1H, H-C(2)); 8.146 (s, 1H, H-C(8)); 7.279 (s, 2H, NH₂); 6.133 (d, 1H, ³J(H-C(1'), H-C(2')) =2.5, H-C(1')); 5.360 *(dd,* 1H, ³J(H-C(2'), H-C(1')) =2.5, ³J(H-C(2'), H-C(3')) =6.0, H-C(2')); 5.102 *(t,* 1H, ³J(HO-C(5'), H₂-C(5')) =5.5, HO-C(5')); 4.964 (dd, 1H, ³J(H-C(3'), H-C(2')) =2.5, ³J(H-C(3'), H-C(4')) =6.0, H-C(3')); 4.204 - 4.178 *(m,* 1H, H-C(4')); 3.545 - 3.473 *(m,* 2H, H₂-C(5')); 1.738- 1.706 *(m,* 2H, H₂C(1a")); 1.558 - 1.543 *(m,* 2H, H₂-C(1b")); 1.429 - 1.420 *(m,* 2H, H₂-C(2a")); 1.297 - 1.237 *(m,* 22H, H₂-C(2b") - H₂-C(7b") + H₂-C(3a") - H₂-C(7a")); 0.873 - 0.820 *(m,* 6H, H₃-C(8a"), H₃-C(8b")). ¹³C-NMR (125.76 MHz, DMSO-*d₆*): 156.025 (C(6)); 152.492 (C(2)); 148.781 (C(4)); 139.584 (C8)); 118.966 (C(5)); 116.626 (C(acetal)); 89.405 (C(1')); 86.809 (C(4')); 83.526 (C(2')); 81.450 (C(3')); 61.503 (C(5')); 36.356 (C(1a")); 36.135 (C(1b")); 31.157 (C(2a")); 31.123 (C(2b")); 30.514 (C(3a")); 29.047 (C(3b")); 28.787 - 28.513 (4 x C, C(4a") - C(7a")); 23.499 - 21.919 (4 x C, C(4b") - C(7b")); 13.778 (C(8a")); 13.746 (C(8b")). ESI - MS: 504.35 [M_{W}+H]⁺ (calculated: M_{W}=503.68). Elemental analysis calculated for C₂₇H₄₅N₅O₄ · 0.05 H₂O · 0.15 Heptadecan-9-one: C, 65.25 (64.38); H, 9.102 (9.01); N, 12.80 (13.90).

### Reference Example 3

### ((2R/2S,3aR,4R,6R,6aR)-6-(6-amino-9H-purin-9-yl)-2-methyl-2-tridecyltetrahydrofuro[3,4-d][1,3]dioxol-4-yl)methanol (2c, NL 5.7₂.0.0, diastereoisomeric mixture, (1R) + (1S))

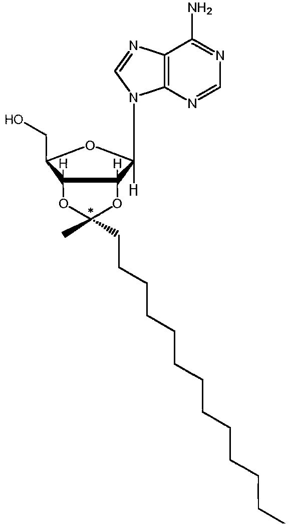

Anhydrous adenosine (1, NS_5.0.0.0; 1.0 g; 3.74 mmol) was dissolved in dry and amine-free dimethylformamide (10 ml). Thereupon, pentadecan-2-one (0.906 g; 4 mmol), triethyl orthoformate (0.662 ml, 4 mmol) and 4M HCl in 1.4-dioxane (4 ml) were added. The reaction mixture was stirred for 24 h at room temperature and then partitioned between CH₂Cl₂ (80 ml) and a saturated, aqueous NaHCO₃ solution (40 ml). The aqueous phase was extracted thrice with CH₂Cl₂ (15 ml, each). The combined organic phases were washed three times with 50 ml H₂O and then dried over MgSO₄ for 30 minutes, filtered and evaporated at a rotary evaporator. Residual DMF was removed by repeated co-evaporation from CH₂Cl₂ and drying in high vacuum overnight. Column chromatography (SiO₂ 60, column: 5 x 7 cm; CH₂Cl₂/MeOH, 9:1 v/v) gave the title compound as a white solid upon evaporation of the main fractions and drying in high vacuum (1.1918 g, 67%).

TLC (SiO₂ 60, CH₂Cl₂/MeOH 9:1; v/v): *R_{f}* 0.80. UV/Vis (MeOH): λ_{ma×}: 260 nm, ε=14260.4 l·mol⁻¹·cm⁻¹. log*P*_{OW}: 2.83. ¹H-NMR (500.13 MHz, DMSO - *d₆*): 8.333 (s, H-C(2), *(1R)* + (1S)); 8.149 (s, H-C(8), (1R) + (1S)); 7.282 (s, NH₂); 6.139 (d, ³*J*(H-C(1'), H-C(2'))=3.0; H-C(1'), (1S)); 6.115 (d, ³*J*(H-C(1'), H-C(2'))=3.0; H-C(1'), (1R)); 5.367 (dd, ³*J*(H-C(2'), H-C(1'))= 3.0; ³*J*(H-C(2'), H-C(3'))=6.5, H-C(2'), (1*R*); 5.287 (*dd*, ³*J*(H-C(2'), H-C(1'))= 3.5; ³*J* (H-C(2'), H-C(3'))= 6.5, HC(2'), (1S); 5.158 - 5.129 *(m,* 2H, HO-C(5'), (1*R*) + *(1S));* 4.980 (*dd*, ³*J*(H-C(3'), H-C(2'))= 2.5; ³*J*(H-C(3'), H-C(4'))=6.5, H-C(3'), (1*R*); 4.947 (*dd*, ³*J*(H-C(3'), H-C(2'))= 2.5; 3*J*(H-C(3'), H-C(4'))=6.0, H-C(3'), (1S); 4.217 - 4.182 *(m,* 1H, H-C(4'), *(1R)* + *(1S));* 3.571 - 3.479 *(m,* 2H, H-C(5'), *(1R)* + *(1S));* 1.766 - 1.734 *(m,* 2H, H2-C(a1), *(1R));* 1.573 - 1.404 *(m,* 5H, H2-C(a1') + H3-C(b1'), (1*S*)); 1.296 - 1.223 (m, 25H, H-C(b1) + H₂-C(a2) - H₂-C(a12), (1R) + H₂-C(a2') - H₂-C(a12'), (1S)); 0.859 - 0.832 *(m,* 3H, H-C(a13) + H-C(a13'), (1R) + (1*S*)). ¹³C-NMR (125.76 MHz, DMSO-d6): 156.049 (C(6), (1*R*) + *(1S));* 152.543 (C(2), (1*R*) + (1S)); 148.819 (C(4), (1*R*) + (1S)); 139.618 (C(8), *(1R)* + *(1S));* 119.006 (C(5), (1*R*) + (1*S*)); 115.018 (C(acetal), (1S)); 114.643 (C(acetal), (1S)); 89.611 (C(1'), (1S)); 89.370 (C(1'), (1R)); 86.628 (C(4'), (1S)); 86.482 (C(4'), (1R)); 83.570 (C(2'), (1S)); 83.140 (C(2'), (1*R*)); 81.607 (C(3'), (1*S*)); 81.105 (C(3'), (1R)); 61.515 (C(5'), (1*R*) *+ (1S));* 31. 199 (C(a11), *(1R)* + *(1S));* 29.060 - 28.587 (10 x C, C(a1) - C(a10), *(1R)* + *(1S));* 23.310 (C(b1), (1R)); 23.180 (C(b1'), (1S)); 21.980 (C(a12), (1*R*) + (1S)); 13.820 (C(a13), *(1R)* + *(1S)).* ESI - MS: 476.32 [M_{W}+H]⁺ (calculated: M_{W}=475.62). Elemental analysis calculated for C₂₅H₄₁N₅O₄ · 0.35 H₂O: C, 62.62 (62.31); H, 8.71 (8.72); N, 14.17 (14.53).

### Reference Example 4

### ((2R/2S,3aR,4R,6R,6aR)-6-(6-amino-9H-purin-9-yl)-2-ethyl-2-tridecyltetrahydrofuro[3,4-d][1,3]dioxol-4-yl)methanol (2d, NL 5.8₃.0.0, diastereoisomeric mixture, (1R) + (1S))

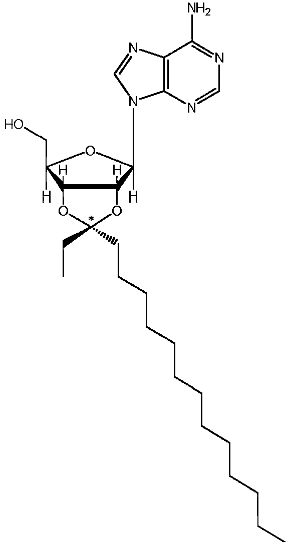

Anhydrous adenosine (1, NS_5.0.0.0; 0.8 g; 2.99 mmol) was dissolved in dry and amine-free dimethylformamide. Thereupon, hexadecan-3-one (1.08 g; 4.5 mmol), triethyl orthoformate (0.745 ml) and 4M HCl in 1.4-dioxane (5 ml) were added. The reaction mixture was stirred for 24 h at room temperature and then partitioned between CH₂Cl₂ (60 ml) and a saturated, aqueous NaHCO₃ solution (40 ml). The aqueous phase was extracted thrice with CH₂Cl₂ (12.5 ml, each). The com bined organic layers were then dried over MgSO₄ (30 min), filtered and evaporated at a rotary evaporator. Residual DMF was removed by repeated co-evaporation from CH₂Cl₂ and drying in high vacuum overnight. Column chromatography (SiO₂ 60, column: 5 x 7 cm; CH₂Cl₂/MeOH, 9:1 v/v) gave the title compound as a slightly white solid upon evaporation of the main fractions and drying in high vacuum (1.0541 g, 72%).

TLC (SiO₂ 60, CH₂Cl₂/MeOH, 9:1; v/v): *R*_{f} 0.81. UV/Vis (MeOH): λ_{ma×}: 260 nm, ε=12434.8 l·mol⁻¹·cm⁻¹. log*P*_{OW}: 3.60. ¹H-NMR (500.13 MHz, DMSO-d₆, 30 °C): 8.331 (s, H-C(2)); 8.147 (s, H-C(8)); 7.279 (s, NH₂)); 6.138 (d, ³*J*(H-C(1'), H-C(2'))=3.0; H-C(1'), (1S)); 6.113 (d, ³*J*(H-C(1'), H-C(2'))=3.0; H-C(1'), (1R)); 5.365 (dd, ³*J*(H-C(2'), H-C(1'))=3.0; ³*J*(H-C(2'), H-C(3'))=6.5, H-C(2'), (1*R*)); 5.328 (dd, ³*J*(H-C(2'), H-C(1'))=3.0; ³*J*(H-C(2'), H-C(3'))=6.5, H-C(2'), (1S)); 5.167 *(t,* ³*J*(HO-C(5'), H₂-C(5'))=5.5, HO-C(5'), (1S)); 5.137 *(t,* ³*J*(HO-C(5'), H₂-C(5'))=5.5, HO-C(5'), (1R)); 4.979 (dd, ³*J*(H-C(3'), H-C(2'))=2.5; ³*J*(H-C(3'), H-C(4'))=6.0, H-C(3'), (1*R*)); 4.946 (dd, ³*J*(H-C(3'), H-C(2'))=2.5; ³*J*(H-C(3'), H-C(4'))=6.0, H-C(3'), (1S)); 4.230 - 4.179 *(m,* 1H, H-C(4'), *(1R)* + *(1S));* 3.571 - 3.512 *(m,* 2H, H₂-C(5'), *(1R) +* (1S)); 1.748 *(dt,* 2H, ²*J*, (H-C(a1"α), (a1"β)=9.0, H₂C(a1"), (1R)); 1.568 *(dt,* 2H, ²*J*, (H-C(a1"α), (a1"β)=10.0, H₂C(a1"), (1S)); 1.500 - 1.400 *(m,* 3H, H₃C(acetal), (1S)); 1.360 - 1.290 *(m,* 3H, H₃C(acetal), (1R)); 1.280 - 1.170 *(m,* 30H, H₂-C(a2"), H₂-C(a3"), H₂-C(a4"), H₂-C(a5"), H₂-C(a6"), H₂-C(a7"), H₂-C(a8"), H₂-C(a9"), H₂-C(a10"), H₂-C(a11"), H₂-C(a12"), H₂-C(a13"), H₂-C(a14"), H₂-C(a15"), H₂-C(a16"), (1R)); 1.280 - 1.170 *(m,* 30H, H₂-C(b2"), H₂-C(b3"), H₂-C(b4"), H₂-C(b5"), H₂-C(b6"), H₂-C(b7"), H₂-C(b8"), H₂-C(b9"), H₂-C(b10"), H₂-C(b11"), H₂-C(b12"), H₂-C(b13"), H₂-C(b14"), H₂-C(b15"), H₂-C(b16"), (1S)); 0.847 (t, ³*J*(H₃-C(17"), H2-C(16")=5.00, (1R) + *(1S)).* ¹³C-NMR (125.8 MHz, 30°C, DMSO-d₆): 156.119 (C(6), (1R)); 156.140 (C(6), (1S)); 152.619 (C(2), (1R) + (1S)); 148.855 (C(4), (1R)); 148.795 (C(4), (1S)); 139.736 (C(8), (1S)); 139.631 (C(8), (1R)); 119.100 (C(5), (1S)); 119.054 (C(5), (1R)); 115.039 (C(acetal), (1S)); 114.656 (C(acetal), (1R)); 89.678 (C(1'), (1S)); 89.433 (C(1'), (1*R*)); 86.635 (C(4'), (1*S*)); 86.539 (C(4'), (1R)); 83.563 (C(2'), (1S)); 83.204 (C(2'), (1R)); 81.672 (C(3'), (1S)); 81.174 (C(3'), (1R)); 61.610 (C(5'), (1S)); 61.569 (C(5'), (1R)); 35.774 (C(a1"), (1R)); 35.730 (C(b1"), (1S)); 31.296 (C(a3"), (1R)); 30.762 (C(b3"), (1S)); 29.174 - 28.711 (11 x C, (C(a4"), (C(a5"), (C(a6"), (C(a7"), (C(a8"), (C(a9"), (C(a10"), (C(a11"), (C(a12"), (C(a13"), (C(a14"), (C(a15"), (1R)); 29.174 - 28.711 (11 x C, (C(b4"), (C(b5"), (C(b6"), (C(b7"), (C(b8"), (C(b9"), (C(b10"), (C(b11"), (C(b12"), (C(b13"), (C(b14"), (C(b15"), (1S)); 25.008 (C(b1"), (1R)); 23.897 (C(a1"), (1S)); 23.351 (C(a2"), (1R)); 23.278 (C(b2"), (1S)); 22.094 (C(a16"), (1R)); 22.040 (C(b16"), (1S)); 13.920 (C(17"), (1R) + *(1S)).* ESI - MS: 532.25 [M_{W}+H]⁺ (calculated: M_{w}=531.73); 605.34 [(M_{w}+DMF)+H]⁺ (calculated: 604.82). Elemental analysis for C₂₉H₄₉N₅O₄ · 0.6 H₂O: C, 63.96 (64.20); H, 9.24 (9.33); N, 13.15 (12.91).

### Example 5

### ((3aR,4R,6R,6aR)-6-(3H-imidazo[2,1-i]purin-3-yl)-2,2-diheptyltetrahydrofuro[3,4-d][1,3]dioxol-4-yl)methanol Hydrochloride (4a, εNL 5.7.0.0).

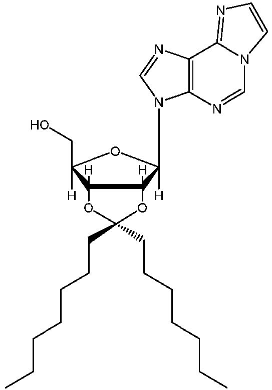

*Synthesis pathway* 1: Anhydrous compound 2c (NL_5.7.0.0, 0.1906 g, 0.4 mmol) was dissolved in 10 ml of a distilled chloroacetaldehyde solution (2 - 3 M). By adding 200 µl of ammonium hydrogen carbonate solution (1 M), the pH value of the reaction mixture was adjusted to 4 - 4.5. The reaction mixture was stirred for 24 h at 37°C. The solvent was concentrated on a rotary evaporator (water bath not above 35°C!) and the residue was then evaporated from ethanol. In a further step, the mixture was distributed between CH₂Cl₂ and an aqueous solution of sodium hydrogen carbonate. Extraction was continued until the aqueous phase hardly fluoresces at all. The combined organic phases were washed with 120 ml of H₂O and then dried for 1 h over magnesium sulfate. The filtered raw product was concentrated at a rotary evaporator. The raw product was dried overnight in high vacuum. The main product was obtained by column chromatography on silica gel (SiO₂ 60, column: 6.5 cm x 5 cm; CH₂Cl₂/MeOH), 93:7 v/v). The isolated and solvent-free product forms a white solid (0.0839 g, 42%) in high vacuum.

*Synthesis pathway 2:* Dry ethenoadenosine (6, εNS_5.0.0.0, 0.174 g; 0.6 mmol) was dissolved in 7 ml of dry and amine-free dimethylformamide. Pentadecan-8-one (0.1585 g; 0.7 mmol), triethyl orthoformate (0.12 ml) and 4M HCl-1.4-dioxane (2 ml) were then added. The solution was stirred for 24 h at room temperature. Then, the reaction mixture was distributed between 60 ml of CH₂Cl₂ and 40 ml of a saturated aqueous solution of sodium hydrogen carbonate. The aqueous phase was extracted with CH₂Cl₂ until the fluorescence had subsided. The combined organic phases were washed with 120 ml of H₂O and then dried for 1 h over magnesium sulfate. The filtered raw product was concentrated at a rotary evaporator. The remaining DMF was evaporated at 30°C by means of a spherical tube apparatus. The main product was obtained by column chromatography on silica gel (SiO₂ 60, column: 6.5 cm x 5 cm; CH₂Cl₂/MeOH), 93:7 v/v). The isolated and solvent-free product forms a white solid in high vacuum (0.0662 g, 22%).

TLC (SiO₂ 60, CH₂Cl₂/MeOH 9:1; v/v): *R_{f}* 0.76. UV/Vis (MeOH): λ_{ma×}: 229 nm, ε=33756.0 l·mol⁻¹·cm⁻¹. Fluorescence (MeOH): λ_{Em}: 408 nm, λ_{Ex}: 233 nm. log*P*_{OW}: 2.40. ¹H-NMR (500.13 MHz, DMSO - *d*₆): 9.296 (s, 1H, H-C(2)); 8.527 (s, 1H, (H-C(8)); 8.090 (d, 1H, ³J(H-C(10),H-C(11) =1.5; H-C(10)); 7.565 (d, 1H, ³J(H-C(11),H-C(10) =1.5; H-C(11)); 6.281 *(d,* 1H, ³J(H-C(1'), H-C(2') =2.0; H-C(1')); 5.395 (*dd*, 1H, ³J(H-C(2'), H-C(1') =2.5; ³J(H-C(2'), H-C(3') =6.5; H-C(2')); 5.043 - 4.986 *(m,* 2H, H-C(3'), H-O(5')); 4.250 - 4.224 *(m,* 1 H, H-C(4')); 3.551 - 3.495 *(m,* 2H, H₂-C(5')); 1.759 - 1.727 *(m,* 2H, H₂-C(1a")); 1.578 - 1.562 *(m,* 2H, H₂-C(1b")); 1.450 - 1.445 *(m,* 2H, H₂-C(2a")); 1.321 - 1.248 *(m,* 18H, H₂-C(2b"), H₂-C(3a"), H₂-C(3b"), H₂-C(4a"-6a"), H₂-C(4b"-6b")); 0.887 - 0.850 *(m,* 6H, H₃-C(7a"), H₃-C(7b")). ¹³C-NMR (125.76 MHz, DMSO-d6): 140.393 (C(6)); 139.911 (C(8)); 137.896 (C(2)); 137.048 (C(4)); 132.762 (C10)); 123.091 (C5)); 116.763 (C(acetal)); 112.164 (C(11)); 89.768 (C(1')); 87.216 (C(4')); 84.136 (C(2')); 81.509 (C(3')); 61.469 (C(5')); 36.339 (C(1a")); 36.288 (C(1b")); 31.156 (C(2a")); 31.103 (C(2b")); 28.644 - 17.412 (C(3a"), C(3b"), C(4a"-6a"), C(4b"-6b")); 13.860 (C(7a")); 13.815 (C(7b")). ESI - MS: 500.26 [M_{w}+H]⁺, 999.47 [2M_{w}+H]⁺ (calculated: M_{w}=499.65). Elemental analysis calculated for C₂₇H₄₁N₅O₄ · 0.55 HCl · 0.1 Pentadecan-8-one: C, 63.23 (63.12); H, 8.094 (8.28); N, 13.02 (12.91).

### Example 6

### ((3aR,4R,6R,6aR)-6-(3H-imidazo[2,1-i]purin-3-yl)-2,2-dioctyitetrahydrofuro[3,4-d][1,3]dioxol-4-yl)methanol Hydrochloride (4b, εNL 5.9.0.0)

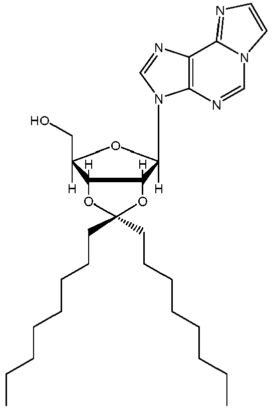

*Synthesis pathway 1:* Dry compound 2d (NL_5.9.0.0, 0.1041 g, 0.21 mmol) was dissolved in 10 ml of a distilled chloroacetaldehyde solution (2 - 3 M). By adding 200 µl of ammonium bicarbonate solution (1 M), the pH value of the reaction mixture was adjusted to 4 - 4.5. The reaction mixture was stirred for 24 h at 37°C. The solvent was concentrated on a rotary evaporator (water bath not above 35°C!) and the residue evaporated several times with ethanol harshly to remove the remaining aqueous chloroacetaldehyde solution. The raw product was dried over night in high vacuum. The main product was obtained by column chromatography on silica gel (SiO₂ 60, column: 8 cm x 5 cm; CH₂Cl₂/MeOH),9:1 v/v). The isolated and solvent-free product forms a white solid (0.065 g, 59%) in high vacuum.

*Synthesis pathway 2:* Anhydrous ethenoadenosine (6, εNS_5.0.0.0, 0.2002 g; 0.69 mmol) was dissolved in 8 ml of dry and amine-free dimethylformamide. Heptadecan-9-one (0.1780 g; 0.7 mmol), triethyl orthoformate (0.16 ml), 4M HCl-1.4-dioxane (4 ml) and CH₂Cl₂ (1.5 ml) were then added. The solution was stirred for 24 h at room temperature. Then, the reaction mixture was distributed between 60 ml of CH₂Cl₂ and 40 ml of a saturated aqueous solution of sodium hydrogen carbonate. The aqueous phase was extracted with CH₂Cl₂ until the fluorescence had subsided. The combined organic phases were dried for 1 h over magnesium sulfate. The filtered raw product was concentrated at a rotary evaporator and was dried overnight in high vacuum. The main product was obtained by column chromatography on silica gel (SiO₂ 60, column: 6.5 cm x 5 cm; CH₂Cl₂/MeOH), 9:1 v/v). The isolated and solvent-free product forms a white solid in high vacuum (0.0874 g, 24%).

TLC (SiO₂ 60, CH₂Cl₂/MeOH 9:1; v/v): *R_{f}* 0.78. UV/Vis (MeOH): λ_{ma×}: 229 nm, ε=28007.2 l·mol⁻¹·cm⁻¹. Fluorescence (MeOH): λ_{Em}: 409 nm, λ_{Ex}: 233 nm. log*P*_{OW}: 2.57. ¹H-NMR (500.13 MHz, DMSO - *d*₆): 9.299 (s, 1H, H-C(2)); 8.529 (s, 1H, (H-C(8)); 8.093 (d, 1H, ³J(H-C(10),H-C(11) =1.5; H-C(10)); 7.565 (*d*, 1H, ³J(H-C(11),H-C(10) =1.5; H-C(11)); 6.281 (*d*, 1H, ³J(H-C(1'), H-C(2') =2.5; H-C(1')); 5.393 (*dd*, 1H, ³J(H-C(2'), H-C(1') =2.5; ³J(H-C(2'), H-C(3') =6.5; H-C(2')); 5.032 (t, 1H, ³J(HO-C(5'), H₂-C(5')) =5.0, HO-C(5')); 5.007 - 4.989 *(m,* 2H, H-C(3'), H-O(5')); 4.250 - 4.224 *(m,* 1H, H-C(4')); 3.563 - 3.498 *(m,* 2H, H₂-C(5')); 1.756 - 1.723 (m, 2H, H₂C(1a")); 1.587 - 1.558 (m, 2H, H₂-C(1b")); 1.460 - 1.416 (m, 2H, H₂-C(2a")); 1.306 - 1.232 (m, 22H, H₂-C(2b") - H₂-C(7b") + H₂-C(3a") - H₂-C(7b")); 0.873 - 0.842 (m, 6H, H₃-C(8a"), H₃-C(8b")). ¹³C-NMR (125.76 MHz, DMSO-*d₆*): 140.350 (C(6)); 139.885 (C(8)); 137.872 (C(2)); 136.999 (C(4)); 132.684 (C10)); 123.058 (C5)); 116.714 (C(acetal)); 112.120 (C(11)); 89.736 (C(1')); 87.188 (C(4')); 84.107 (C(2')); 81.464 (C(3')); 61.432 (C(5')); 36.306 (C(1a")); 36.210 (C(1b")); 31.161 (C(2a")); 31.129 (C(2b")); 30.521 (C(3a")); 29.057 (C(3b")); 28.802 - 28.462 (4 x C, C(4a") - C(7a")); 23.516 - 21.926 (4 x C, C(4b") - C(7b")); 13.800 (C(8a")); 13.765 (C(8b")). ESI - MS: 528.35 [M_{w}+H]⁺, 1055.7 [2M_{w}+H]⁺ (calculated: M_{w}=527.70). Elemental analysis calculated for C₂₉H₄₅N₅O₄ · 0.35 HCl · 0.5 H₂O · 0.05 Pentadecan-8-one: C, 63.79 (63.77); H, 8.71 (8.61); N, 12.37 (12.46).

### Example 7

### ((2R/2S,3aR,4R,6R,6aR)-6-(6-amino-9H-purin-9-yl)-2-methyl-2-tridecyltetrahydrofuro[3,4-d][1,3]dioxol-4-yl)methanol Hydrochloride (4c, εNL 5.7₂.0.0, diastereoisomeric mixture, (1R) + (1S))

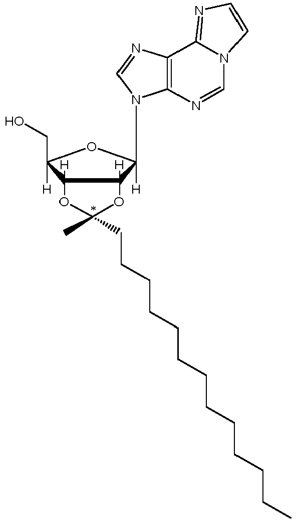

*Synthesis pathway* 1: Anhydrous compound 2h (NL_5.7₂.0.0, 0.1154 g, 0.24 mmol) was dissolved in 10 ml of a distilled chloroacetaldehyde solution (2 - 3 M). By adding 200 µl of ammonium hydrogen carbonate solution (1 M), the pH value of the reaction mixture was adjusted to 4 - 4.5. The reaction mixture was stirred for 24 h at 37°C. The solvent was concentrated on a rotary evaporator (water bath not above 35°C!) and the residue evaporated several times with ethanol harshly to remove the remaining aqueous chloroacetaldehyde solution. The raw product was dried over night in high vacuum. The main product was obtained by column chromatography on silica gel (SiO₂ 60, column: 8 cm x 5 cm; CH₂Cl₂/MeOH),9:1 v/v). The isolated and solvent-free product forms a white solid (0.072 g, 60%) in high vakuum.

*Synthesis pathway 2:* Dry ethenoadenosine (6, εNS_5.0.0.0, 0.2002 g; 0.69 mmol) was dissolved in 8 ml of dry and amine-free dimethylformamide and 1 ml of distilled CH₂Cl₂. Pentadecan-2-one (0.1573 g; 0.7 mmol), triethyl orthoformate (0.16 ml) and 4M HCl-1.4-dioxane (3 ml) were then added. The solution was stirred for 24 h at room temperature. Then, the reaction mixture was distributed between 60 ml of CH₂Cl₂ and 40 ml of a saturated aqueous solution of sodium hydrogen carbonate. The aqueous phase was extracted with CH₂Cl₂ until the fluorescence had subsided. The combined organic phases were washed with 100 ml of H₂O and then dried for 1 h over magnesium sulphate. The filtered raw product was concentrated at a rotary evaporator. The remaining DMF was evaporated at 30°C by means of a spherical tube apparatus. The main product was obtained by column chromatography on silica gel (SiO₂ 60, column: 9.0 cm x 5 cm; CH₂Cl₂/MeOH), 9:1 v/v). The isolated and solvent-free product forms a white solid in high vacuum (0.1378 g, 40%).

TLC (SiO₂ 60, CH₂Cl₂/MeOH 9:1; v/v): *R_{f}* 0.76. UV/Vis (MeOH): λₘₐₓ: 229 nm, ε=30843.8 l·mol⁻¹·cm⁻¹. Fluorescence (MeOH): λ_{Em}: 408 nm, λ_{Ex}: 235 nm. log*P*_{OW}: 2.75. ¹H-NMR (500.13 MHz, DMSO - *d*₆): 9.300 (s, H-C(2), (1*R*) + *(1S));* 8.525 (s, H-C(8), (1*R*) *+* (1*S*)); 8.089 (*d*, ³*J*(H-C(10), H-C(11)=1.5; H-C(10), (1*R*) + (1*S*)); 7.566 (*d*, ³*J*(H-C(11), H-C(11) =1.5; H-C(10), *(1R)* + *(1S));* 6.289 (d, ³*J*(H-C(1'), H-C(2'))=2.5; H-C(1'), (1S)); 6.277 (d, ³*J*(H-C(1'), HC(2'))=2.5; H-C(1'), (1R)); 5.402 (dd, ³*J*(H-C(2'), H-C(1'))= 3.0; ³J(H-C(2'), H-C(3'))=6.5, H-C(2'), (1R); 5.364 (dd, ³J(H-C(2'), H C(1'))=2.5; ³J(H-C(2'), H-C(3'))= 6.0, HC(2'), (1S); 5.050 (t, ³*J*(HO-C(5'), H₂-C(5'))=5.0, HO-C(5'), (1S) + (1R)); 5.011 (dd, ³J(H-C(3'), H-C(2'))= 3; ³J(H-C(3'),H-C(4'))= 6.5, H-C(3'), (1R); 4.983 (dd, ³J(H-C(3'), H-C(2'))= 2.5; ³J(H-C(3'), H-C(4'))=6.0, H-C(3'), (1S); 4.252 - 4.227 *(m,* 1H, H-C(4'), (1R) + *(1S));* 3.578 - 3.504 *(m,* 2H,H₂-C(5'), *(1R)* + (1*S*)); 1.787 - 1.755 *(m,* 2H, H₂-C(1a"), (1R)); 1.606-1.443 *(m,* 5H, H₂-C(1b") + H₃-C(1a"), (1S)); 1.298 - 1.217 (m, 25H, H₃-C(1b") + H₂-C(2a") - H₂-C(12a"), (1R) + H₂-C(2b") - H₂-C(12b"), (1S)); 0.855 - 0.828 *(m,* 3H, H₃-C(13a"), *(1R) +* H-C(13b"), (1S)). ¹³C-NMR (125.76 MHz, DMSO-d6): 140.363 (C(6), (1R) + (1S)); 139.867 (C(8), (1S)); 139.801 (C(8), (1R)); 137.855 (C(2), (1*R*) + (1S)); 137.010 (C(4), (1*R*) + *(1S));* 132.732 (C(10), *(1R)* + (1S)); 123.101 (C(5), (1R) + (1S)); 115.088 (C(acetal), (1R)); 114.675 (C(acetal), (1S)); 112.110 (C(11), (1*R*) + (1*S*)); 89.857 (C(1'), (1S)); 89.685 (C(1'), (1*R*)); 86.978 (C(4'), (1S)); 86.858 (C(4'), (1R)); 84.139 (C(2'), (1S)); 83.718 (C(2'), (1R)); 81.569 (C(3'), (1S)); 81.083 (C(3'), (1*R*)); 61.408 (C(5'), (1*R*) + (1S)); 39.220 (C(1a"), (1R)); 38.203 (C(1b"), (1S)); 31. 163 (C(11a"), *(1R)* + C(11b") *(1S));* 29.030 - 28.570 (9 x C, C(2a") - C(10a"), *(1R)* + C(2b") - C(10b"), *(1S));* 23.334 (C(1b"), (1R)); 23.132 (C(1a"), (1S)); 21.952 (C(12a"), *(1R)* + C(12b"), (1*S*)); 13.793 (C(12a"), *(1R)* + C(13b"), (1S)). ESI - MS: 500.32 [M_{w}+H]⁺, 999.64 [2M+H]⁺ (calculated: M_{w}=499.65). Elemental analysis calculated for C₂₇H₄₁N₅O₄ · 0.3 HCl · 0.05 H₂O · 0.1 Pentadecan-2-one: C, 64.09 (64.09); H, 8.33 (8.38); N, 13.06 (13.11).

### Example 8

### ((2R/2S,3aR,4R,6R,6aR)-6-(3H-imidazo[2,1-i]purin-3-yl)-2-ethyl-2-tridecyltetrahydrofuro[3,4-d][1,3]dioxol-4-yl)methanol Hydrochloride (4d, εNL 5.8₃.0.0, diastereoisomeric mixture, (1R) + (1S))

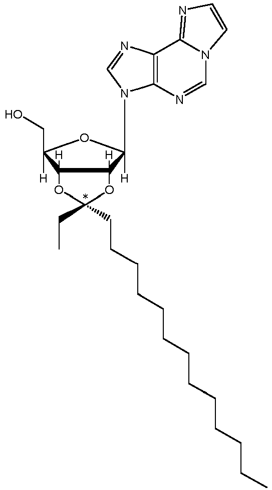

Synthesis pathway 1: Anhydrous compound 2i (NL_5.8₃.0.0, 0.130 g, 0.45 mmol) was dissolved in a distilled chloroacetaldehyde solution (2-3 M, 10 ml). The pH of the reaction mixture was adjusted to 4 - 4.5 by addition of 200 µl of a 1M ammonium hydrogen carbonate - solution. After stirring of this solution for 24 h at 37°C the solvent was removed at a rotary evaporator with a water bath below 35 °C, and the residue was co-evaporated from EtOH. Next, the raw product was partitioned between CH₂Cl₂ and an aqueous NaHCO₃ solution. The extraction was repeated until the aqueous phase shows no more fluorescence. The combined organic layers were dried over MgSO₄ (30 min), filtered and evaporated at a rotary evaporator. The residue was further dried overnight in high vacuum. Column chromatography (SiO₂ 60, column: 5.5 x 4.5 cm; CH₂Cl₂/MeOH, 9:1 v/v) gave the title compound after evaporation of the appropriate fractions and forms a white solid (0.1109 g, 48%).

Synthesis pathway 2: Anhydrous 1,N⁶-ethenoadenosine (6, εNS_5.0.0.0, 0.173 g, 0.594 mmol) was dissolved in dry and amine-free dimethylformamide (5 ml). Subsequently, hexa-decan-3-one (0.168 g; 0.7 mmol), triethyl orthoformate (0.12 ml) and 4M HCl in 1.4-dioxane (1.5 ml) were added. The mixture was stirred for 24 h at room temperature and then partitioned between CH₂Cl₂ (60 ml) and a saturated aqueous NaHCO₃ solution (40 ml). The aqueous phase was extracted repeatedly until the fluorescence had ceased. The combined organic layers were evaporated on a rotary evaporator; residual DMF was removed with a spherical tube apparatus at 30°C. Column chromatography (SiO₂ 60, column: 5.5 x 4.5 cm; CH₂Cl₂/MeOH, 9:1 v/v) gave the title compound as a white solid after evaporation of the appropriate fractions and drying in high vacuum (0.1067 g, 35%).

TLC (SiO₂ 60, CH₂Cl₂/MeOH 9:1; v/v): *R_{f}* 0.77. UV/Vis (MeOH): λ_{ma×}: 229 nm, ε=36445.9 l·mol⁻¹·cm⁻¹. Fluorescence (MeOH): λ_{Em}: 408 nm, λ_{Ex}: 235 nm. log*P*_{OW}: 2.29.¹H-NMR (500.13 MHz, DMSO-*d₆*, 30°C): 9.304 (s, H-C(2), (1*R*)); 9.299 (s, H-C(2), (1*S*)); 8.533 (s, H-C(8), (1R)); 8.527 (s, H-C(8), (1S)); 8.088 (d, ³*J*(H-C(11), H-C(11)=1.0; H-C(10), (1R) *+* (1*S*)); 7.564 (*d*, ³*J*(H-C(11), H-C(10)=1.5; H-C(10), (1*R*) + (1*S*)); 6.292 (*d*, ³*J*(H-C(1'), H-C(2'))=2.0; H-C(1'), (1*R*) + (1*S*)); 5.403 (*dd*, ³*J*(H-C(2'), H-C(1'))=2.5; ³*J*(H-C(2'), H-C(3')=6.0; H-C(2'), (1*R*) + (1S)); 5.039 *(t,* ³*J*(HO-C(5'), H₂-C(5')=5.0, HO-C(5'), (1*R*) + (1S); 5.013 - 4.994 *(m,* 1H, H-C(3'), *(1R)* + *(1S));* 4.266 - 4.234 *(m,* 1H, H-C(4'), *(1R)* + *(1S));* 3.574 - 3.489 *(m,* 2H, H₂-C(5'), (1R) + (1S)); 1.794 - 1.720 *(m,* 2H, H₂-C(1a") + H₂-C(1b"), (1R)); 1.628 - 1.563 *(m,* 2H, H₂-C(1b") + H₂-C(1a"), (1S)); 1.463 - 1.428 *(m,* 2H, H₂-C (2b"), *(1S));* 1.342 - 1.294 *(m,* 2H, H₂-C (2a"), (1R)); 1.284 - 1.170 (m, 23H, H₂-C(3a") - H₂-C(12a"), (1R) + H₂-C(3b") - H₂-C(12b"), (1S)); 0.977 *(t,* ³*J*(H₃-C(2b"), H₂-C(b1")=7.5, (1R)); 0.848 *(t,* ³*J*(H₃-C(a2"), H₂-C(a1")=3.5, (1S)); 0.837 *(t,* ³*J*(H₃-C(13a"), H₂-C(12a")=2.5, (1R)); 0.821 *(t,* ³*J*(H₃-C(13b"), H₂-C(12b")=2.5, *(1S)).* ¹³C-NMR (125.8 MHz, 30°C, DMSO-*d₆*): 140.374 (C(6), *(1R)* + *(1S));* 139.892 (C(8), (1R)); 139.862 (C(8), (1*S*)); 137.856 (C(4), *(1R)* + *(1S));* 137.018 (C(2), (1R)); 136.996 (C(2), (1*S*)); 132.733 (C(10), *(1R)* + *(1S));* 123.095 (C(5), *(1R) + (1S));* 117.020 (C(acetal), (1S)); 116.918 (C(acetal), (1R)); 112.100 (C(11), *(1R)* + *(1S));* 89.794 (C(1'), (1*R*) + (1S)); 87.233 (C(4'), (1*S*)); 87.148 (C(4'), (1R)); 84.187 (C(2'), (1*S*)); 84.164 (C(2'), (1R)); 81.540 (C(3'), (1R) + (1S)); 61.441 (C(5'), (1R) + (1S)); 36.054 (C(1a"), (1R)); 35.774 (C(1b"), (1S)); 31.161 (C(11a"), (1R) + C(11b"), (1S)); 29.102 - 28.558 (C(3a"), (C(4a"), (C(5a"), (C(6a"), (C(7a"), (C(8a"), (C(9a"), (C(10a"), (1R)); 29.102 - 28.558 (C(3b"), (C(4b"), (C(5b"), (C(6b"), (C(7b"), (C(8b"), (C(9b"), (C(10b"), (1S)); 23.506 (C(2a"), (1R)); 22.818 (C(2b"), (1*S*)); 21.952 (C(12a"), (1R) + (C(12b"), (1S)); 13.779 (C(2b"), (1R) + (C(2a"), (1S)); 8.230 (C(13b"), (1S)); 7.595 (C(13a"), (1R)). ESI - MS: 514.25 [M_{w}+H]⁺, 1027.51 [2M+H]⁺ (calculated: M_{w}=513.67). Elemental analysis calculated for C₂₈H₄₃N₅O₄ · 0.2 HCl · 0.05 H₂O · 0.05 Hexadecan-3-one: C, 64.75 (64.79); H, 8.39 (8.48); N, 13.20 (13.12).

### Example 9

### ((3a'R,4'R,6'R,6a'R)-4'-(3H-imidazo[2,1-i]purin-3-yl)tetrahydrospiro[cyclopentadecane-1,2'-furo[3,4-d][1,3]dioxol]-6'-yl)methanol Hydrochloride (5, εNL 5.cycl7.0.0)

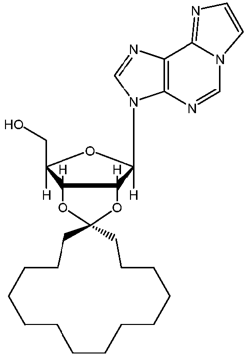

*Synthesis pathway 1:* Dry compound 3 (NL_5.cycl7.0.0, 0.1271 g, 0.26 mmol) was dissolved in 10 ml of a distilled chloroacetaldehyde solution (2 - 3 M). By adding 200 µl of ammonium bicarbonate solution (1 M), the pH value oft the reaction mixture was adjusted to 4 - 4.5. The reaction mixture was stirred for 24 h at 37°C. The solvent was concentrated on a rotary evaporator (water bath not above 35°C!) and the residue evaporated several times with ethanol harshly to remove the remaining aqueous chloroacetaldehyde solution. The raw product was dried over night in high vacuum. The main product was obtained by column chromatography on silica gel (SiO₂ 60, column: 7 cm x 5 cm; CH₂Cl₂/MeOH),9:1 v/v). The isolated and solvent-free product forms a yellowish oil (0.072 g, 55%) in high vacuum.

*Synthesis pathway 2:* Anhydrous ethenoadenosine (6, εNS_5.0.0.0, 0.2000 g; 0.69 mmol) was dissolved in 8 ml of dry and amine-free dimethylformamide. Cyclopentadecanone (0.1564 g; 0.7 mmol), triethyl orthoformate (0.16 ml), 4M HCl-1.4-dioxane (3 ml) and CH₂Cl₂ (0.5 ml) were then added. The solution was stirred for 24 h at room temperature. Then, the reaction mixture was distributed between 60 ml of CH₂Cl₂ and 40 ml of a saturated aqueous solution of sodium hydrogen carbonate. The aqueous phase was extracted with CH₂Cl₂ until the fluorescence had subsided. The combined organic phases were dried for 1 h over magnesium sulfate. The filtered raw product was concentrated at a rotary evaporator and was dried overnight in high vacuum. The main product was obtained by column chromatography on silica gel (SiO₂ 60, column: 6.5 cm x 5 cm; CH₂Cl₂/MeOH), 9:1 v/v). The isolated and solvent-free product forms a yellowish oil in high vacuum (0.1172 g, 34%).

TLC (SiO₂ 60, CH₂Cl₂/MeOH 9:1; v/v): *R_{f}* 0.76. UV/Vis (MeOH): λ_{ma×}: 229 nm, ε=30631.1 l·mol⁻¹·cm⁻¹. Fluorescence (MeOH): λ_{Em}: 409 nm, λ_{Ex}: 234 nm. log*P*_{OW}:1.67. ¹H-NMR (500.13 MHz, DMSO - *d*₆): 9.305 (s, H-C(2); 8.522 (s, H-C(8)); 8.088 (d, ³*J*(H-C(10), H-C(11)=1.5; H-C(10)); 7.566 (d, ³*J*(H-C(11), H-C(11) =1.5; H-C(10)); 6.269 (d, ³*J*(H-C(1'), H-C(2'))=2.5; H-C(1')); 5.383 (dd, ³*J*(H-C(2'), H-C(1'))= 3.0; ³J(H-C(2'), H-C(3'))=6.5, H-C(2')); 5.036 (t, ³*J*(HO-C(5'), H₂-C(5'))=5.5, HO-C(5')); 4.980 (dd, ³J(H-C(3'), H-C(2'))= 3.0; ³J(H-C(3'),H-C(4'))= 6.5, H-C(3')); 4.246 - 4.221 *(m,* 1H, H-C(4')); 3.577 - 3.499 (*m*, 2H,H2-C(5')); 1.805 - 1.775 (m, H₂-C(1a")); 1.610 - 1.582 (m, H₂C(14a")); 1.414 - 1.303 (m, 24H, H₂-C(2a") - H₂-C(13a")). ¹³C-NMR (125.76 MHz, DMSO-d*₆*): 140.350 (C(6)); 139.930 (C(8)); 137.854 (C(2)); 137.034 C(4)); 132.702 (C10)); 123.099 (C5)); 116.841 (C(acetal)); 112.126 (C(11)); 89.745 (C(1')); 86.934 (C(4')); 83.646 (C(2')); 81.134 (C(3')); 61.416 (C(5')); 36.464 (C(1a")); 34.179 (C(14a")); 26.895 (C(2a")); 26.855 (C(13a")); 26.324 (C(3a")); 26.306 (C(12a")); 26.138 - 21.977 (8 x C, C(4a") - C(11a")). ESI - MS: 498.31 [M_{w}+H]⁺, 995.6 [2M_{w}+H]⁺ (calculated: M_{w}=497.63)^{,} Elemental analysis calculated for C₂₇H₃₉N₅O₄ · 0.2 HCl: C, 64.24 (64.23); H, 7.93 (7.83); N, 13.77 (13.87).

### Example 10

### Determination of activity against human and murine cancer cells

Of the compounds described in examples 1 to 9, the cytotoxic effect on different cell lines was determined *in vitro.* The cell lines were rat malignant neuroectodermal BT4Ca and human GOS3 glioma cells, human U87, U251, G112 and G28 glioblastoma cells. In addition, the cytotoxic effect on human HT29 colon adenocarcinoma cells, human HepG2 hepatocellular carcinoma cells, murine RenCa renal carcinoma cells and human Panc-1 pancreatic carcinoma cells were investigated. The compounds are soluble in DMSO, which was therefore also used as a negative control. The nucleoside 5-fluorouridine (5-FUrd) was used as a positive control, except for the glioblastoma cell lines. In these, the currently used chemotherapeutic agent temozolomide (TMZ) was the positive control. All compounds were applied at six different concentrations (1.56 µM, 3.12 µM, 6.25 µM, 12.5 µM, 25 µM and 50 µM). The cells were treated during 48h with the adenosine and ethenoadenosine derivatives or the positive controls. Subsequently, viability was determined by Presto Blue^{®} assay. Each experiment was performed independently four times and the results were expressed as mean ± standard error of the mean (SEM). Depending on the mode of distribution, statistical procedures were performed by the Mann-Whitney U-Wilcoxon Rank Sum W-test or by the Student's t-test for unpaired data. A p value of 0.05 or less was taken for statistical significance. The results are shown in figures 1 to 22.

**Figure 1** shows the viability (in % of the negative control) of rat BT4Ca glioma cells after 48 h incubation with 5-FUrd (positive control) or the derivatives NL_5.7.0.0, NL_5.7₂.0.0, NL_5.8₃.0.0 and NL_5.9.0.0 measured by PrestoBlue^{®} assay. Values are given (in % viability of control [incubation with medium alone; = 100 % viability]) as mean ± SEM; *p≤0.05, **p≤0.01, ***p≤0.001, significance vs. 0.25 % DMSO control; n=4 independent experiments. assayed in quadruplicates.

**Figure 2** shows the viability (in % of the negative control) of rat BT4Ca glioma cells after 48 h incubation with 5-FUrd (positive control) or the derivatives εNL_5.7.0.0, εNL_5.7₂.0.0, εNL_5.8₃.0.0, εNL_5.9.0.0 and εNL_5.cycl7.0.0 measured by PrestoBlue^{®} assay. Values are given (in % viability of control [incubation with medium alone; = 100 % viability]) as mean ± SEM; *p≤0.05,**p≤0.01, ***p≤0.001, significance vs. 0.25 % DMSO control; n=4 independent experiments assayed in quadruplicates.

**Figure 3** shows the Viability (in % of the negative control) of human GOS3 glioma cells after 48 h incubation with 5-FUrd (positive control) or the derivatives NL_5.7.0.0, NL_5.7₂.0.0, NL_5.8₃.0.0 and NL_5.9.0.0 measured by PrestoBlue^{®} assay. Values are given (in % viability of control [incubation with medium alone; = 100 % viability]) as mean ± SEM; *p≤0.05, **p≤0.01, **p≤0.001, significance vs. 0.25 % DMSO control; ^{#}p≤0.05, ^{##}p≤0.01, ^{###}p≤0.001, significance vs. 5-FUrd at equal concentration; n=4 independent experiments assayed in quadruplicates.

**Figure 4** shows the viability (in % of the negative control) of human GOS3 glioma cells after 48 h incubation with 5-FUrd (positive control) or the derivatives εNL_5.7.0.0, εNL_5.7₂.0.0, εNL_5.8₃.0.0, εNL_5.9.0.0 and εNL_5.cycl7.0.0 measured by PrestoBlue^{®} assay. Values are given (in % viability of control [incubation with medium alone; = 100 % viability]) as mean ± SEM; *p≤0.05,**p≤0.01, ***p≤0.001, significance vs. 0.25 % DMSO control; ^{#}p≤0.05, ^{##}p≤0.01, ^{###}p≤0.001,significance vs. 5-FUrd at equal concentration; n=4 independent experiments assayed in quadruplicates.

**Figure 5** shows the viability (in % of the negative control) of human U87 glioblastoma cells after 48 h incubation with TMZ (positive control) or the derivatives NL_5.7.0.0, NL_5.7₂.0.0, NL_5.8₃.0.0 and NL_5.9.0.0 measured by PrestoBlue^{®} assay. Values are given (in % viability of control [incubation with medium alone; = 100 % viability]) as mean ± SEM; *p≤0.05, **p≤0.01, ***p≤0.001, significance vs. 0.25 % DMSO control; ^{#}p≤0.05, ^{##}p≤0.01, ^{###}p≤0.001, significance vs. TMZ at equal concentration; n=4 independent experiments assayed in quadruplicates.

**Figure 6** shows the viability (in % of the negative control) of human U87 glioblastoma cells after 48 h incubation with TMZ (positive control) or the derivatives εNL_5.7.0.0, εNL_5.7₂.0.0, εNL_5.8₃.0.0, εNL_5.9.0.0 and εNL_5.cycl7.0.0 measured by PrestoBlue^{®} assay. Values are given (in % viability of control [incubation with medium alone; = 100 % viability]) as mean ± SEM; *p≤0.05,**p≤0.01, ***p≤0.001, significance vs. 0.25 % DMSO control; ^{#}p≤0.05, ^{##}p≤0.01, ^{###}p≤0.001 significance vs. TMZ at equal concentration; n=4 independent experiments assayed in quadruplicates.

**Figure 7** shows the viability (in % of the negative control) of human U251 glioblastoma cells after 48 h incubation with TMZ (positive control) or the derivatives NL_5.7.0.0, NL_5.7₂.0.0, NL_5.8₃.0.0 and NL_5.9.0.0 measured by PrestoBlue^{®} assay. Values are given (in % viability of control [incubation with medium alone; = 100 % viability]) as mean ± SEM; *p≤0.05, **p≤0.01, ***p≤0.001, significance vs. 0.25 % DMSO control; ^{#}p≤0.05, ^{##}p≤0.01, ^{###}p≤0.001 significance vs. TMZ at equal concentration; n=4 independent experiments. assayed in quadruplicates.

**Figure 8** shows the viability (in % of the negative control) of human U251 glioblastoma cells after 48 h incubation with TMZ (positive control) or the derivatives εNL_5.7.0.0, εNL_5.7₂.0.0, εNL_5.8₃.0.0, εNL_5.9.0.0 and εNL_5.cycl7.0.0 measured by PrestoBlue^{®} assay. Values are given (in % viability of control [incubation with medium alone; = 100 % viability]) as mean ± SEM; *p≤0.05, **p≤0.01, ***p≤0.001, significance vs. 0.25 % DMSO control; ^{#}p≤0.05, ^{##}p≤0.01, ^{###}p≤0.001 significance vs. TMZ at equal concentration; n=4 independent experiments

**Figure 9** shows the viability (in % of the negative control) of human G28 glioblastoma cells after 48 h incubation with TMZ (positive control) or the derivatives NL_5.7.0.0, NL_5.7₂.0.0, NL_5.8₃.0.0 and NL_5.9.0.0 measured by PrestoBlue^{®} assay. Values are given (in % viability of control [incubation with medium alone; = 100 % viability]) as mean ± SEM; *p≤0.05, **p≤0.01, ***p≤0.001, significance vs. 0.25 % DMSO control; ^{#}p≤0.05, ^{##}p≤0.01, ^{###}p≤0.001 significance vs. TMZ at equal concentration; n=4 independent experiments assayed in quadruplicates.

**Figure 10** shows the viability (in % of the negative control) of human G28 glioblastoma cells after 48 h incubation with TMZ (positive control) or the derivatives εNL_5.7.0.0, εNL_5.7₂.0.0, εNL_5.8₃.0.0, εNL_5.9.0.0 and εNL_5.cycl7.0.0 measured by PrestoBlue^{®} assay. Values are given (in % viability of control [incubation with medium alone; = 100 % viability]) as mean ± SEM; *p≤0.05,**p≤0.01, ***p≤0.001, significance vs. 0.25 % DMSO control; ^{#}p≤0.05, ^{##}p≤0.01, ^{###}p≤0.001 significance vs. TMZ at equal concentration; n=4 independent experiments assayed in quadruplicates.

**Figure 11** shows the viability (in % of the negative control) of human G112 glioblastoma cells after 48 h incubation with TMZ (positive control) or the derivatives NL_5.7.0.0, NL_5.7₂.0.0, NL_5.8₃.0.0 and NL_5.9.0.0 measured by PrestoBlue^{®} assay. Values are given (in % viability of control [incubation with medium alone; = 100 % viability]) as mean ± SEM; *p≤0.05, **p≤0.01, ***p≤0.001, significance vs. 0.25 % DMSO control; ^{#}p≤0.05, ^{##}p≤0.01, ^{###}p≤0.001 significance vs. TMZ at equal concentration; n=4 independent experiments assayed in quadruplicates.

**Figure 12** shows the viability (in % of the negative control) of human G112 glioblastoma cells after 48 h incubation with TMZ (positive control) or the derivatives εNL_5.7.0.0, εNL_5.7₂.0.0, εNL_5.8₃.0.0, εNL_5.9.0.0 and εNL_5.cycl7.0.0 measured by PrestoBlue^{®} assay. Values are given (in % viability of control [incubation with medium alone; = 100 % viability]) as mean ± SEM; *p≤0.05,**p≤0.01, ***p≤0.001, significance vs. 0.25 % DMSO control; ^{###}p≤0.001 significance vs. TMZ at equal concentration; n=4 independent experiments assayed in quadruplicates.

**Figure 13** shows the viability (in % of the negative control) of human HT29 colon adenocarcinoma cells after 48 h incubation with 5-FUrd (positive control) or the derivatives NL_5.7.0.0, NL_5.7₂.0.0, NL_5.8₃.0.0 and NL_5.9.0.0 measured by PrestoBlue^{®} assay. Values are given (in % viability of control [incubation with medium alone; = 100 % viability]) as mean ± SEM; *p≤0.05,**p≤0.01, ***p≤0.001, significance vs. 0.25 % DMSO control; ^{##}p≤0.01, ^{###}p≤0.001 significance vs. 5-FUrd at equal concentration; n=4 independent experiments assayed in quadruplicates.

**Figure 14** shows the viability (in % of the negative control) of rat human HT29 colon adenocarcinoma cells after 48 h incubation with 5-FUrd (positive control) or the derivatives εNL_5.7.0.0, εNL_5.7₂.0.0, εNL_5.8₃.0.0, εNL_5.9.0.0 and εNL_5.cycl7.0.0 measured by PrestoBlue^{®} assay. Values are given (in % viability of control [incubation with medium alone; = 100 % viability]) as mean ± SEM; *p≤0.05,**p≤0.01, ***p≤0.001, significance vs. 0.25 % DMSO control; ^{###}p≤0.001 significance vs. 5-FUrd at equal concentration; n=4 independent experiments assayed in quadruplicates.

**Figure 15** shows the viability (in % of the negative control) of human HepG2 hepatocellular carcinoma cells after 48 h incubation with 5-FUrd (positive control) or the derivatives NL_5.7.0.0, NL_5.7₂.0.0, NL_5.8₃.0.0 and NL_5.9.0.0 measured by PrestoBlue^{®} assay. Values are given (in % viability of control [incubation with medium alone; = 100 % viability]) as mean ± SEM; *p≤0.05,**p≤0.01, ***p≤0.001, significance vs. 0.25 % DMSO control; ^{#}p≤0.05, ^{##}p≤0.01, ^{###}p≤0.001 significance vs. 5-FUrd at equal concentration; n=4 independent experiments assayed in quadruplicates.

**Figure 16** shows the viability (in % of the negative control) of human HepG2 hepatocellular carcinoma cells after 48 h incubation with 5-FUrd (positive control) or the derivatives εNL_5.7.0.0, εNL_5.7₂.0.0, εNL_5.8₃.0.0, εNL_5.9.0.0 and εNL_5.cycl7.0.0 measured by PrestoBlue^{®} assay. Values are given (in % viability of control [incubation with medium alone; = 100 % viability]) as mean ± SEM; **p≤0.01, ***p≤0.001, significance vs. 0.25 % DMSO control; ^{#}p≤0.05, ^{##}p≤0.01, significance vs. 5-FUrd at equal concentration; n=4 independent experiments assayed in quadruplicates.

**Figure 17** shows the viability (in % of the negative control) of murine RenCa renal carcinoma cells after 48 h incubation with 5-FUrd (positive control) or the derivatives NL_5.7.0.0, NL_5.7₂.0.0, NL_5.8₃.0.0 and NL_5.9.0.0 measured by PrestoBlue^{®} assay. Values are given (in % viability of control [incubation with medium alone; = 100 % viability]) as mean ± SEM; ***p≤0.001, significance vs. 0.25 % DMSO control; ^{#}p≤0.05, ^{###}p≤0.001, significance vs. 5-FUrd at equal concentration; n=4 independent experiments assayed in quadruplicates.

**Figure 18** shows the viability (in % of the negative control) of murine RenCa renal carcinoma cells after 48 h incubation with 5-FUrd (positive control) or the derivatives εNL_5.7.0.0, εNL_5.7₂.0.0, εNL_5.8₃.0.0, εNL_5.9.0.0 and εNL_5.cycl7.0.0 measured by PrestoBlue^{®} assay. Values are given (in % viability of control [incubation with medium alone; = 100 % viability]) as mean ± SEM; **p≤0.01 ***p≤0.001, significance vs. 0.25 % DMSO control; ^{##}p≤0.01, ^{###}p≤0.001 significance vs. 5-FUrd at equal concentration; n=4 independent experiments assayed in quadruplicates.

**Figure 19** shows the viability (in % of the negative control) of human Panc-1 pancreatic carcinoma cells after 48 h incubation with 5-FUrd (positive control) or the derivatives NL_5.7.0.0, NL_5.7₂.0.0, NL_5.8₃.0.0 and NL_5.9.0.0 measured by PrestoBlue^{®} assay. Values are given (in % viability of control [incubation with medium alone; = 100 % viability]) as mean ± SEM; **p<0.01, ***p≤0.001, significance vs. 0.25 % DMSO control; ^{##}p≤0.01, ^{###}p≤0.001 significance vs. 5-FUrd at equal concentration; n=4 independent experiments assayed in quadruplicates.

**Figure 20** shows the viability (in % of the negative control) of human Panc-1 pancreatic carcinoma cells after 48 h incubation with 5-FUrd (positive control) or the derivatives εNL_5.7.0.0, εNL_5.7₂.0.0, εNL_5.8₃.0.0, εNL_5.9.0.0 and εNL_5.cycl7.0.0 measured by PrestoBlue^{®} assay. Values are given (in % viability of control [incubation with medium alone; = 100 % viability]) as mean ± SEM; *p≤0.05, **p≤0.01, ***p≤0.001, significance vs. 0.25 % DMSO control; ^{###}p≤0.001 significance vs. 5-FUrd at equal concentration; n=4 independent experiments assayed in quadruplicates.

The data visualized in Figures 1 to 20 show that the compounds tested have cytotoxic effects on different types of cancer cells such as e. g. glioma, glioblastoma, colon adenocarcinoma, hepatocellular carcinoma, renal carcinoma, and pancreatic carcinoma. They have pronounced cytostatic/cytotoxic activity towards different cancer entities of various species *in vitro.* They are more effective than the known anti-cancer drug TMZ, even at concentrations 10 times lower, and more effective than 5-FU that never reached 100% cytotoxicity at any concentration tested.

### Example 11

### Determination of activity against pancreatic ductal adenocarcinoma (PDAC)

The biological antitumor-activity of adenosine derivatives according to Formula I towards pancreatic ductal adenocarcinoma (PDAC) was evaluated by utilizing the established cell line Panc-1 as a PDAC model by applying the CellTiter-Glo^{®} Luminescent Cell Viability Assay (Promega) after 72 h of compound incubation. Gemcitabine (100 µM) was used as a positive control. To analyze the cytotoxic efficacy in Panc-1 cells, dose-response curves were generated for each compound, starting from a concentration of 100 µM with serial dilution of 1:3. IC₅₀ values are shown as arithmetic means of at least three independent biological repeats in following Table 1.

**Table 1: IC₅₀-Values for the inhibition of PADC by adenosine and 1,N⁶-ethenoadenosine derivatives (with the respective standard deviations SD)**

| **Compound** | **IC₅₀-Value [µM] ± SD** |
|---|---|
| **2a** (NL_5.7.0.0) | 14.58 ± 0.14 |
| **2d** (NL_5.8₃.0.0) | 14.58 ± 0.69 |
| **4a** (εNL_5.7.0.0) | 17.08 ± 3.14 |
| **4b** (εNL_5.9.0.0) | 16.18 ± 1.35 |
| **4c** (εNL 5.7₂.0.0) | 30.64 ± 2.53 |
| **4d** (εNL_5.8₃.0.0) | 15.95 ± 4.31 |
| **5** (εNL_5.cycl7.0.0) | 23.13 ± 3.60 |

## Claims

1. An adenosine derivative of Formula I, or a pharmaceutically acceptable salt thereof: 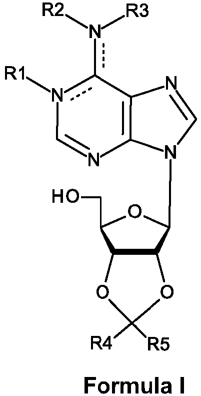 wherein:
R¹ and R² together form an ethenylene group, the bond between N¹ and C⁶ is a single bond, the bond between C⁶ and the exocyclic amino nitrogen is a double bond, and R³ is absent, and
R⁴ and R⁵ are each independently a branched or preferably linear C₁-C₁₆-alkyl group, provided that R⁴ and R⁵ together comprise 11 to 17 carbon atoms, or R⁴ and R⁵ together with the carbon atom to which they are bound form a monocyclic or polycyclic cycloaliphatic ring with 10 to 15 carbon atoms.

2. The adenosine derivative of claim 1, wherein
R⁴ and R⁵ are each independently a branched or preferably linear C₁-C₁₅-alkyl group, provided that R⁴ and R⁵ together comprise 11 to 16 carbon atoms, or R⁴ and R⁵ together with the carbon atom to which they are bound form a monocyclic ring with 10 to 15 carbon atoms or a polycyclic cycloaliphatic ring with 10 to 15 carbon atoms.

3. The adenosine derivative of claim 2, wherein
R⁴ and R⁵ are each independently a branched or preferably linear C₁-C₁₃-alkyl group, provided that R⁴ and R⁵ together comprise 11 to 16 carbon atoms, or R⁴ and R⁵ together with the carbon atom to which they are bound form a monocyclic ring with 10 to 14 carbon atoms or a polycyclic cycloaliphatic ring with 10 to 15 carbon atoms.

4. The adenosine derivative of claim 1, wherein:
both R⁴ and R⁵ are each a linear C₆-C₈ alkyl group, preferably a C₇ to C₈ alkyl group, or one of R⁴ and R⁵ is a linear C₁-C₃-alkyl group, preferably C₁-C₂-alkyl group, and the other is a linear C₈-C₁₆ alkyl group, preferably C₁₀-C₁₅ alkyl group, more preferably a C₁₃ alkyl group.

5. The adenosine derivative of claim 4, having the Formula II: 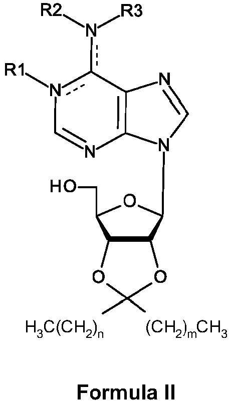 wherein:
n and m are each an integer of 0 to 15, preferably 0 to 14, more preferably 0 to 12, provided that the sum of m and n is in a range of from 9 to 15, preferably 10 to 14, preferably 11 to 14 and most preferably 12 to 14.

6. The adenosine derivative of claim 5, wherein m=n= 6 or m=n=7 or n=0 and m=12 or n=1 and m=12.

7. The adenosine derivative of claim 1, wherein R⁴ and R⁵ together with the carbon atom to which they are bound form a polycyclic or preferably monocyclic cycloaliphatic ring with 10 to 15 carbon atoms, preferably 10 to 14 carbon atoms.

8. The adenosine derivative of claim 7, having the Formula III: 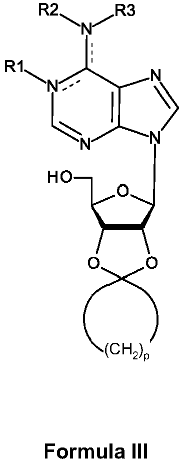 wherein p is an integer within the range of from 9 to 14, preferably 10 to14 and most preferably 10 to 13.

9. The adenosine derivative according to any one of claims 1 to 8, or a pharmaceutically acceptable salt thereof, for use as a medicament.

10. The adenosine derivative, or a pharmaceutically acceptable salt thereof, for use according to claim 9 for the therapy of cancer.

11. The adenosine derivative, or a pharmaceutically acceptable salt thereof, for use in the therapy of cancer according to claim 10, wherein the cancer is brain cancer, colon cancer, liver cancer, renal cancer or pancreatic cancer.

12. The adenosine derivative, or a pharmaceutically acceptable salt thereof, for use in the therapy of cancer according to claim 11, wherein the cancer is a glioma and especially a glioblastoma, or a carcinoma.

13. The adenosine derivative, or a pharmaceutically acceptable salt thereof, for use in the therapy of cancer according to claim 11, wherein the cancer is an adenocarcinoma of the colon or pancreas, a pancreatic ductal adenocarcinoma, a hepatocellular carcinoma, a renal cell carcinoma or a pancreatic carcinoma.

14. Pharmaceutical composition for use as a medicament for the therapy of cancer, comprising a pharmaceutically effective amount of at least one adenosine derivative according to any one of claim 1 to 8, or a pharmaceutically acceptable salt thereof.

15. The pharmaceutical composition for use in the therapy of cancer according to claim 14, comprising a pharmaceutically effective amount of at least one adenosine derivative according to any one of claim 1 to 8, or a pharmaceutically acceptable salt thereof, in the form of a dry powder and water for injection for the reconstitution of the powder.

## Patentansprüche

1. Adenosinderivat der Formel I oder ein pharmazeutisch verträgliches Salz davon: 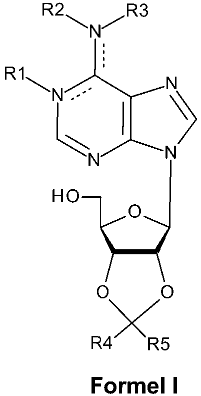 wobei:
R¹ und R² zusammen eine Ethenylengruppe bilden, die Bindung zwischen N¹ und C⁶ eine Einfachbindung ist, die Bindung zwischen C⁶ und dem exocyclischen Aminostickstoff eine Doppelbindung ist, und R³ fehlt, und
R⁴ und R⁵ jeweils unabhängig voneinander eine verzweigte oder bevorzugt lineare C₁-C₁₆-Alkylgruppe sind, vorausgesetzt, dass R⁴ und R⁵ zusammen 11 bis 17 Kohlenstoffatome umfassen, oder R⁴ und R⁵ zusammen mit dem Kohlenstoffatom, an das sie gebunden sind, einen monocyclischen oder polycyclischen cycloaliphatischen Ring mit 10 bis 15 Kohlenstoffatomen bilden.

2. Das Adenosinderivat nach Anspruch 1, wobei
R⁴ und R⁵ jeweils unabhängig voneinander eine verzweigte oder bevorzugt lineare C₁ -C₁₅-Alkylgruppe sind, vorausgesetzt, dass R4 und R5 zusammen 11 bis 16 Kohlenstoffatome umfassen, oder R⁴ und R⁵ zusammen mit dem Kohlenstoffatom, an das sie gebunden sind, einen monocyclischen Ring mit 10 bis 15 Kohlenstoffatomen oder einen polycyclischen cycloaliphatischen Ring mit 10 bis 15 Kohlenstoffatomen bilden.

3. Das Adenosinderivat nach Anspruch 2, wobei
R⁴ und R⁵ jeweils unabhängig voneinander eine verzweigte oder bevorzugt lineare C₁ -C₁₃-Alkylgruppe sind, vorausgesetzt, dass R⁴ und R⁵ zusammen 11 bis 16 Kohlenstoffatome umfassen, oder R⁴ und R⁵ zusammen mit dem Kohlenstoffatom, an das sie gebunden sind, einen monocyclischen Ring mit 10 bis 14 Kohlenstoffatomen oder einen polycyclischen cycloaliphatischen Ring mit 10 bis 15 Kohlenstoffatomen bilden.

4. Das Adenosinderivat nach Anspruch 1, wobei:
sowohl R⁴ als auch R⁵ jeweils eine lineare C₆-C₈-Alkylgruppe, bevorzugt eine C₇- bis C₈-Alkylgruppe, sind oder eines von R⁴ und R⁵ eine lineare C₁-C₃-Alkylgruppe, bevorzugt eine C₁-C₂-Alkylgruppe, ist und der andere eine lineare C₈-C₁₆-Alkylgruppe, bevorzugt eine C₁₀-C₁₅-Alkylgruppe, weiter bevorzugt eine C₁₃-Alkylgruppe ist.

5. Das Adenosinderivat nach Anspruch 4 mit der Formel II: 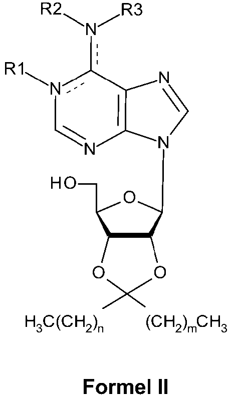 wobei:
n und m sind jeweils eine ganze Zahl von 0 bis 15, bevorzugt von 0 bis 14, weiter bevorzugt von 0 bis 12, vorausgesetzt, dass die Summe von m und n in einem Bereich von 9 bis 15, bevorzugt von 10 bis 14, bevorzugt von 11 bis 14 und besonders bevorzugt von 12 bis 14 liegt.

6. Das Adenosinderivat nach Anspruch 5, wobei m = n = 6 oder m = n = 7 oder n = 0 und m = 12 oder n = 1 und m = 12.

7. Das Adenosinderivat nach Anspruch 1, wobei R⁴ und R⁵ zusammen mit dem Kohlenstoffatom, an das sie gebunden sind, einen polycyclischen oder bevorzugt monocyclischen cycloaliphatischen Ring mit 10 bis 15 Kohlenstoffatomen, bevorzugt 10 bis 14 Kohlenstoffatomen, bilden.

8. Das Adenosinderivat nach Anspruch 7 mit der Formel III: 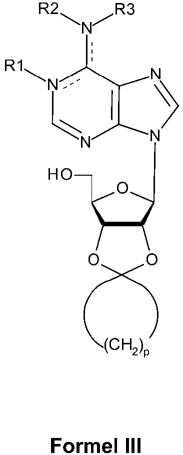 wobei p eine ganze Zahl im Bereich von 9 bis 14, bevorzugt 10 bis 14 und weiter bevorzugt 10 bis 13 ist.

9. Adenosinderivat gemäß einem der Ansprüche 1 bis 8 oder ein pharmazeutisch verträgliches Salz davon zur Verwendung als Arzneimittel.

10. Das Adenosinderivat oder ein pharmazeutisch verträgliches Salz davon zur Verwendung gemäß Anspruch 9 zur Therapie von Krebs.

11. Das Adenosinderivat oder ein pharmazeutisch verträgliches Salz davon zur Verwendung bei der Therapie von Krebs gemäß Anspruch 10, wobei es sich bei dem Krebs um Hirntumor, Darmkrebs, Leberkrebs, Nierenkrebs oder Bauchspeicheldrüsenkrebs handelt.

12. Das Adenosinderivat oder ein pharmazeutisch verträgliches Salz davon zur Verwendung in der Therapie von Krebs gemäß Anspruch 11, wobei es sich bei dem Krebs um ein Gliom und insbesondere ein Glioblastom oder ein Karzinom handelt.

13. Das Adenosinderivat oder ein pharmazeutisch verträgliches Salz davon zur Verwendung in der Therapie von Krebs gemäß Anspruch 11, wobei es sich bei dem Krebs um ein Adenokarzinom des Dickdarms oder der Bauchspeicheldrüse, ein duktales Adenokarzinom der Bauchspeicheldrüse, ein Leberzellkarzinom, ein Nierenzellkarzinom oder ein Pankreaskarzinom handelt.

14. Pharmazeutische Zusammensetzung zur Verwendung als Medikament zur Therapie von Krebs, umfassend eine pharmazeutisch wirksame Menge mindestens eines Adenosinderivats gemäß einem der Ansprüche 1 bis 8 oder eines pharmazeutisch akzeptablen Salzes davon.

15. Pharmazeutische Zusammensetzung zur Verwendung bei der Therapie von Krebs gemäß Anspruch 14, umfassend eine pharmazeutisch wirksame Menge mindestens eines Adenosinderivats gemäß einem der Ansprüche 1 bis 8 oder eines pharmazeutisch akzeptablen Salzes davon in Form eines Trockenpulvers und Wasser zur Injektion zur Rekonstitution des Pulvers.

## Revendications

1. Dérivé d'adénosine de formule I ou un sel pharmaceutiquement acceptable de celui-ci: 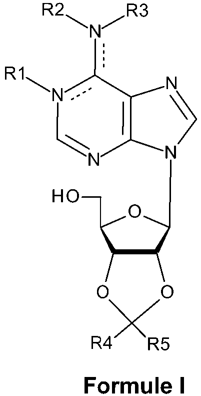 dans lequel:
R¹ et R² forment ensemble un groupe éthénylène, la liaison entre N¹ et C⁶ est une liaison simple, la liaison entre C⁶ et l'azote amino exocyclique est une double liaison, et R³ est absent, et
R⁴ et R⁵ sont chacun indépendamment un groupe alkyle en C₁-C₁₆ ramifié ou de préférence linéaire, à condition que R⁴ et R⁵ comprennent ensemble 11 à 17 atomes de carbone, ou bien R⁴ et R⁵, conjointement avec l'atome de carbone auquel ils sont liés, forment un cycle cycloaliphatique monocyclique ou polycyclique ayant de 10 à 15 atomes de carbone.

2. Dérivé d'adénosine selon la revendication 1, dans lequel
R⁴ et R⁵ sont chacun indépendamment un groupe alkyle en C₁ -C₁₅ ramifié ou de préférence linéaire, à condition que R⁴ et R⁵ contiennent ensemble 11 à 16 atomes de carbone, ou bien R⁴ et R⁵, conjointement avec l'atome de carbone auquel ils sont liés, forment un cycle monocyclique ayant 10 à 15 atomes de carbone ou un cycle cycloaliphatique polycyclique ayant 10 à 15 atomes de carbone.

3. Dérivé d'adénosine selon la revendication 2, dans lequel
R⁴ et R⁵ sont chacun indépendamment un groupe alkyle en C₁ -C₁₃ ramifié ou de préférence linéaire, à condition que R⁴ et R⁵ contiennent ensemble 11 à 16 atomes de carbone, ou bien R⁴ et R⁵, conjointement avec l'atome de carbone auquel ils sont liés, forment un cycle monocyclique ayant 10 à 14 atomes de carbone ou un cycle cycloaliphatique polycyclique ayant 10 à 15 atomes de carbone.

4. Dérivé d'adénosine selon la revendication 1, dans lequel:
R⁴ et R⁵ sont tous deux un groupe alkyle linéaire en C₆-C₈, de préférence un groupe alkyle en C₇-C₈, ou l'un des groupes R⁴ et R⁵ est un groupe alkyle linéaire en C₁-C₃, de préférence un groupe alkyle en C₁-C₂, et l'autre est un groupe alkyle linéaire en C₈-C₁₆, de préférence un groupe alkyle en C₁₀-C₁₅, de préférence encore un groupe alkyle en C₁₃.

5. Dérivé d'adénosine selon la revendication 4, répondant à la formule II: 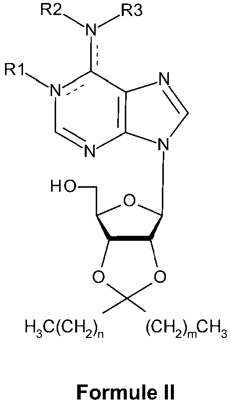 dans lequel:
n et m sont chacun un nombre entier compris entre 0 et 15, de préférence entre 0 et 14, plus préférentiellement entre 0 et 12, à condition que la somme de m et n soit comprise dans une plage de 9 à 15, de préférence de 10 à 14, de préférence de 11 à 14 et de préférence particulière de 12 à 14.

6. Dérivé d'adénosine selon la revendication 5, dans lequel m = n = 6 ou m = n = 7 ou n = 0 et m = 12 ou n = 1 et m = 12.

7. Dérivé d'adénosine selon la revendication 1, dans lequel R⁴ et R⁵, conjointement avec l'atome de carbone auquel ils sont liés, forment un cycle cycloaliphatique polycyclique ou de préférence monocyclique comportant 10 à 15 atomes de carbone, de préférence 10 à 14 atomes de carbone.

8. Dérivé d'adénosine selon la revendication 7, répondant à la formule III: 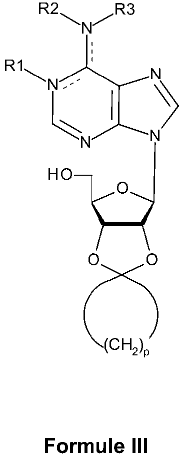 dans lequel p est un nombre entier compris entre 9 et 14, de préférence entre 10 et 14, et plus préférentiellement encore entre 10 et 13.

9. Dérivé d'adénosine selon l'une des revendications 1 à 8 ou un sel pharmaceutiquement acceptable de celui-ci destiné à être utilisé comme médicament.

10. Dérivé d'adénosine ou sel pharmaceutiquement acceptable de celui-ci, destiné à être utilisé selon la revendication 9 pour le traitement du cancer.

11. Dérivé d'adénosine ou sel pharmaceutiquement acceptable de celui-ci destiné à être utilisé dans le traitement du cancer selon la revendication 10, dans lequel le cancer est une tumeur cérébrale, un cancer colorectal, un cancer du foie, un cancer du rein ou un cancer du pancréas.

12. Dérivé d'adénosine ou sel pharmaceutiquement acceptable de celui-ci destiné à être utilisé dans le traitement du cancer selon la revendication 11, dans lequel le cancer est un gliome et en particulier un glioblastome ou un carcinome.

13. Dérivé d'adénosine ou sel pharmaceutiquement acceptable de celui-ci destiné à être utilisé dans le traitement du cancer selon la revendication 11, dans lequel le cancer est un adénocarcinome du côlon ou du pancréas, un adénocarcinome canalaire du pancréas, un carcinome hépatocellulaire, un carcinome rénal ou un carcinome pancréatique.

14. Composition pharmaceutique destinée à être utilisée comme médicament pour le traitement du cancer, comprenant une quantité pharmaceutiquement efficace d'au moins un dérivé d'adénosine selon l'une des revendications 1 à 8 ou d'un sel pharmaceutiquement acceptable de celui-ci.

15. Composition pharmaceutique destinée à être utilisée dans le traitement du cancer selon la revendication 14, comprenant une quantité pharmaceutiquement efficace d'au moins un dérivé d'adénosine selon l'une des revendications 1 à 8 ou d'un sel pharmaceutiquement acceptable de celui-ci sous forme d'une poudre sèche et de l'eau pour injection pour reconstituer la poudre.
